(19) 
Europäisches
Patentamt
European
Patent Office
Office européen
des brevets

(11)  **EP 3 939 603 A1**

(12)  **EUROPEAN PATENT APPLICATION**
published in accordance with Art. 153(4) EPC

(43) Date of publication:
**19.01.2022  Bulletin 2022/03**

(21) Application number: **20769598.2**

(22) Date of filing: **28.02.2020**

(51) International Patent Classification (IPC):
*A61K 36/82* (2006.01)    *A61K 31/192* (2006.01)
*A61K 31/353* (2006.01)    *A61P 3/04* (2006.01)
*A61P 21/00* (2006.01)    *A23L 33/105* (2016.01)
*A23L 29/00* (2016.01)

(52) Cooperative Patent Classification (CPC):
**A23L 29/00; A23L 33/105; A61K 31/192;
A61K 31/353; A61K 36/82; A61P 3/04; A61P 21/00**

(86) International application number:
**PCT/KR2020/002940**

(87) International publication number:
**WO 2020/184879 (17.09.2020 Gazette 2020/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **12.03.2019  KR 20190028377**

(71) Applicant: **Bionic Trading Corporation
Gyeonggi-do 15588 (KR)**

(72) Inventors:
• **KIM, Tae Young**
**Ansan-si, Gyeonggi-do 15463 (KR)**
• **MOON, Joo Myung**
**Ansan-si, Gyeonggi-do 15449 (KR)**

• **KYONG, Su Hyun**
**Anyang-si, Gyeonggi-do 14055 (KR)**
• **LEE, Jae Kyoung**
**Cheonan-si, Chungcheongnam-do 31112 (KR)**
• **KIM, Hyung Joong**
**Cheongju-si, Chungcheongbuk-do 28123 (KR)**
• **KIM, Yoon Hee**
**Ansan-si, Gyeonggi-do 15572 (KR)**
• **CHA, Kyu Min**
**Ansan-si, Gyeonggi-do 15535 (KR)**
• **KIM, Dong Hyeon**
**Ansan-si, Gyeonggi-do 15628 (KR)**
• **KOO, Na Yeon**
**Ansan-si, Gyeonggi-do 15347 (KR)**

(74) Representative: **Gassner, Birgitta et al
REDL Life Science Patent Attorneys
Donau-City-Straße 11
1220 Wien (AT)**

(54) **CATECHIN ENZYME-TREATED MATERIAL HAVING INCREASED GALLIC ACID,
EPICATECHIN AND EPIGALLOCATECHIN CONTENTS, AND METHOD FOR PREPARING
SAME**

(57)    The present invention relates to a method for producing an enzymatically treated catechin product with increased contents of gallic acid, epicatechin (EC), and epigallocatechin (EGC), an enzymatically treated catechin product produced by way of the method, and use thereof.

**EP 3 939 603 A1**

FIG. 2

## Description

### Technical Field

The present invention relates to a method for producing an enzymatically treated catechin product with increased contents of gallic acid, epicatechin (EC), and epigallocatechin (EGC), an enzymatically treated catechin product produced by way of the method, and use thereof.

### Background Art

*Camella sinensis L.*, which is a perennial evergreen shrub belonging to the Camellia family, was first cultivated in China and India, and since then has spread to Southeast Asia, such as to Java, Sri Lanka, Myanmar, and Thailand, and East Asia, such as to Korea and Japan, and is mainly cultivated in Asia below 35 degrees north latitude.

Young leaves from *Camella sinensis L.* are collected and dried to a moisture content of 5% or less, and then divisionally used as white tea, black tea, green tea, and the like according to the color resulting from oxidization during extraction of the leaves. In Korea, more than about 2.5 million tons of green tea plants are grown every year in natural environments with an average annual temperature of 15°C or higher and an average annual rainfall of 1,500 mm or more, such as the valleys of Jirisan, the Seomjin river basin, and the Seogwipo region of Jeju island.

One of the main active ingredients in such tea is a catechin, which is a polyphenol-based compound. Specifically, the main catechin ingredients of green tea are epigallocatechin gallate (EGCG), epicatechin gallate (ECG), epigallocatechin (EGC), epicatechin (EC), and gallic acid.

Catechins have been known to have cholesterol elevation inhibitory action, $\alpha$-amylase activity inhibitory action, or the like (Korean Patent Publication No. 10-2007-0019395 and Korean Patent Registration No. 10-0891393). It has been reported that epicatechin (EC) is effective in the treatment and prevention of senile muscular diseases (Korean Patent Publication No. 10-2018-0009938), epigallocatechin (EGC) has anti-oxidative activity (Korean Patent Publication No. 10-2012-0021407), and gallic acid has excellent effects in anti-oxidation, skin whitening, moisturizing, wrinkle prevention, and relief, and is especially effective in the treatment and prevention of obesity (Anjali Pandey et al. Advances in Research 2(10):556-570, 2014).

Epigallocatechin gallate (EGCG) and epicatechin gallate (ECG) have disadvantages of generating bitter and astringent tastes together with tannins, causing precipitation in a solubilized state, and inhibiting digestive enzymes, and to remedy such disadvantages, it is necessary to convert EGCG and ECG into EGC and EC by using an enzyme.

Most enzymes have their activity inhibited by reaction products produced after reactions in order to maintain homeostasis. However, a particular enzyme has its activity inhibited due to a high concentration of reaction substrate, wherein the activity inhibition is known to occur due to the competitive binding of substrates to a binding site of the enzyme. In such cases, for the prevention of activity inhibition caused by the substrates, the concentration of the enzyme is increased, or the volume of the reaction solution is increased.

However, the conventional art had problems in that processes take a long time, and gallate-degrading enzymes, such as tannase, have activity inhibition occurring even at a low concentration of reaction substrates, and thus there is no method capable of commercially treating high concentrations of substrates.

### Disclosure

### Technical Problem

The present inventors made intensive efforts to derive a method for stably degrading EGCG and ECG as catechins without inactivation of enzymes, and as a result, they have identified that by continuously repeating a step of adding a catechin solution to a tannase solution and a step of reacting a catechin and tannase without the addition of a catechin solution, enzymatic reactions are completed in a short time without the inhibition of enzyme activity while the total amount of substrate usable in the reactions is increased, leading to mass-production of an enzymatically treated catechin product with reduced contents of EGCG and ECG and increased contents of gallic acid, EC, and EGC, thereby completing the present invention.

### Technical Solution]

An object of the present invention is to provide a method for producing an enzymatically treated catechin product, the method including: a first step of preparing a tannase solution; a second step of adding a catechin solution to the tannase solution; a third step of reacting catechins and tannase contained in the solutions in the second step; and a fourth step of continuously repeating the second step and the third step.

Another aspect of the present invention is to provide an enzymatically treated catechin product containing less than 1 part by weight of the sum of epigallocatechin gallate (EGCG) and epicatechin gallate (ECG) relative to 100 parts by weight of the sum of gallic acid, epigallocatechin gallate (EGCG), epicatechin gallate (ECG), epigallocatechin (EGC), and epicatechin (EC).

Still another aspect of the present invention is to provide a pharmaceutical composition for preventing or treating obesity or sarcopenia, the composition containing the enzymatically treated catechin product.

Still another aspect of the present invention is to provide a food composition for preventing or alleviating obesity or sarcopenia, the composition containing the enzymatically treated catechin product.

Still another aspect of the present invention is to provide use of the enzymatically treated catechin for preventing or treating obesity or sarcopenia.

Still another aspect of the present invention is to provide a method for preventing or treating obesity or sarcopenia in a subject in need thereof, the method including administering to the subject the enzymatically treated catechin product.

**Advantageous Effects**

According to the present invention, an enzymatically treated catechin product with reduced contents of EGCG and ECG can be produced by continuously repeating a step of adding a catechin solution to a tannase solution and a step of reacting catechins and tannase without the addition of the catechin solution. The use of the production method can complete enzyme reactions without the inhibition of enzyme activity in a short time, and therefore, the present invention can be widely utilized in large-scale processes for producing useful products by treating catechins with tannase. Furthermore, the enzymatically treated catechin product has increased contents of gallic acid, EC, and ECG, and thus is effective in the prevention or treatment of obesity or sarcopenia.

**Brief Description of Drawings**

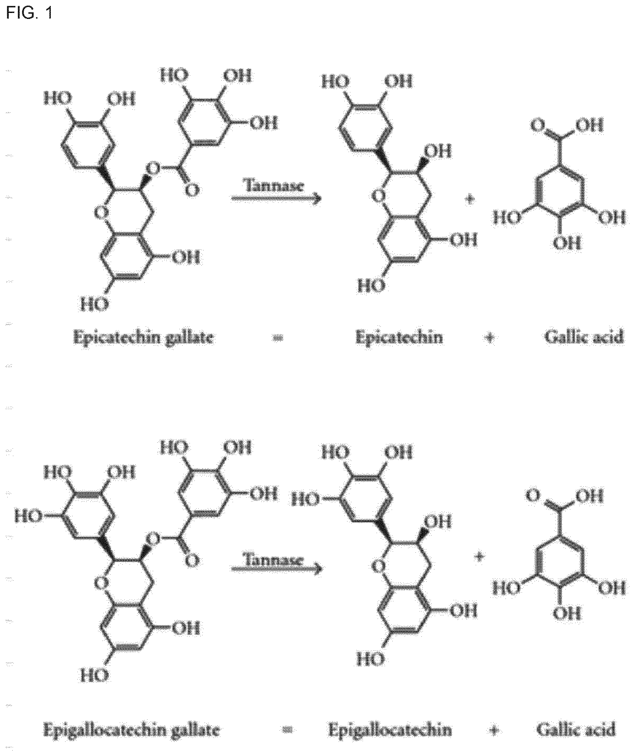

FIG. 1 shows the conversion of green tea catechin ingredients through tannase treatment.

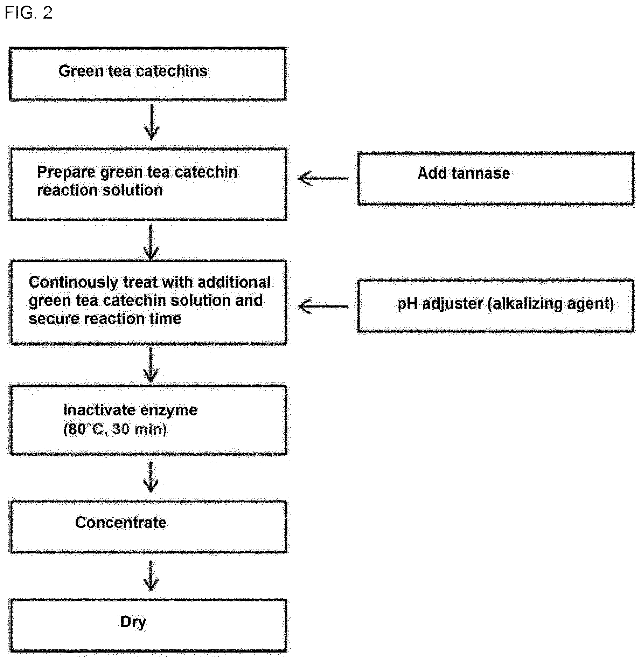

FIG. 2 is a schematic diagram showing a process for producing an enzymatically treated green tea catechin product.

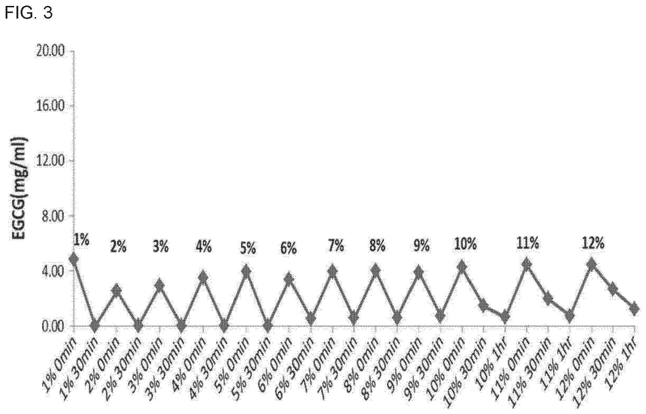

FIG. 3 is a graph showing the results of continuously adding a catechin powder in an amount of 1% such that the total amount of catechins added was 12%, at the concentration of tannase compared with a solution being 4 units, wherein the content of EGCG in the reaction solution of catechins and tannase was measured every 30 minutes. Each numerical value (%) on the graph indicates the amount of catechins accumulated.

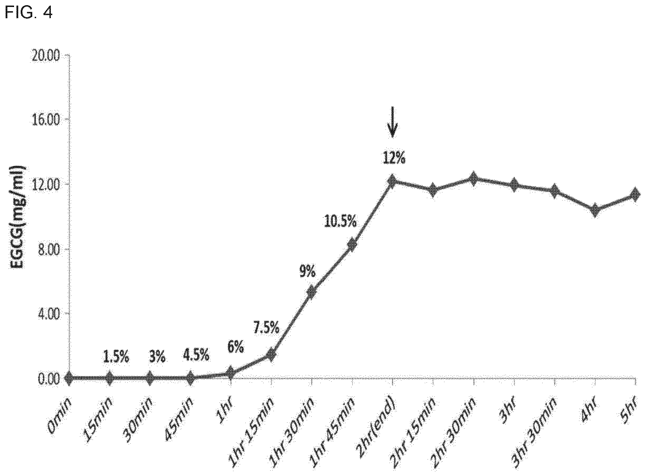

FIG. 4 is a graph showing the results of continuously adding a catechin solution at 8.3 mL per minute such that the amount of catechins added per minute was 0.1% (3 g of catechins/3000 mL), and the total amount of catechins added was 12% (360 g of catechins/3000 mL), at the concentration of tannase compared with the solution being 4 units, wherein the content of EGCG in the reaction solution of catechins and tannase was measured every 15 minutes. Each numerical value (%) on the graph indicates the amount of catechins accumulated.

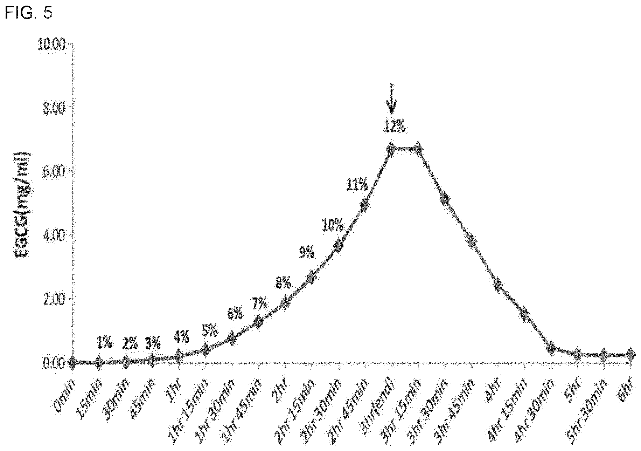

FIG. 5 is a graph showing the results of continuously adding a catechin solution at 6 mL per minute such that the amount of catechins added per minute was 0.07% (2 g of catechins/3000 mL), and the total amount of catechins added was 12% (360 g of catechins/3000 mL), at the concentration of tannase compared with the solution being 4 units, wherein the content of EGCG in the reaction solution of catechins and tannase was measured every 15 minutes. Each numerical value (%) on the graph indicates the amount of catechins accumulated.

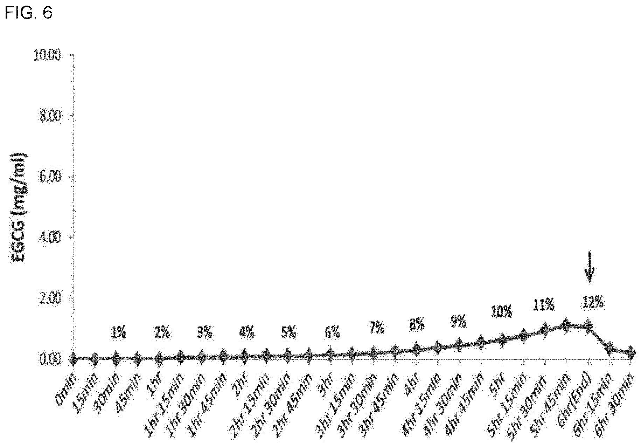

FIG. 6 is a graph showing the results of repeating the procedure of: adding a catechin solution at 8.3 mL per minute for 5 minutes such that the amount of catechins added per minute was 0.1% (3 g of catechins/3000 mL), and the total amount of catechins added was 12% (360 g of catechins/3000 mL), at the concentration of tannase compared with the solution being 4 units; and carrying out a reaction for 10 minutes, wherein the content of EGCG in the reaction solution of catechins and tannase was measured every 15 minutes. Each numerical value (%) on the graph indicates the amount of catechins accumulated.

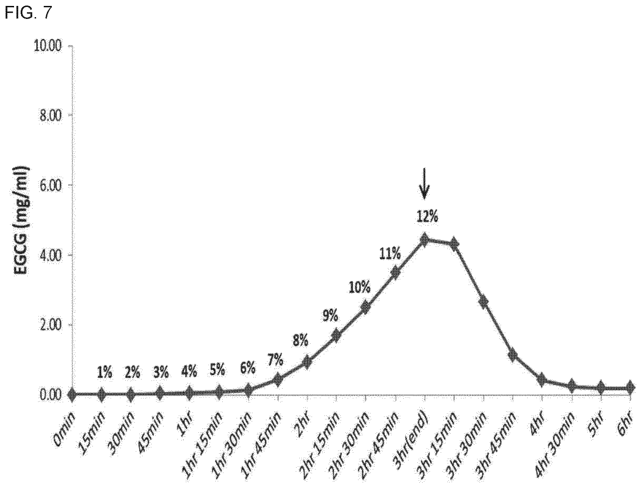

FIG. 7 is a graph showing the results of repeating the procedure of: adding a catechin solution at 8.3 mL per minute for 10 minutes such that the amount of catechins added per minute was 0.1% (3 g of catechins/3000 mL), and the total amount of catechins added was 12% (360 g of catechins/3000 mL), at the concentration of tannase compared with the solution being 4 units; and carrying out a reaction for 5 minutes, wherein the content of EGCG in the reaction solution of catechins and tannase was measured every 15 minutes. Each numerical value (%) on the graph indicates the amount of catechins accumulated.

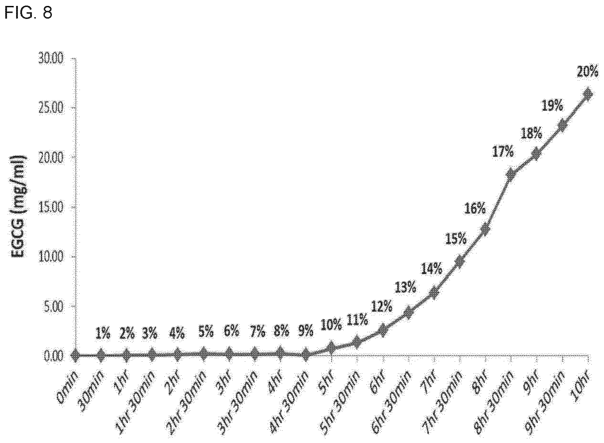

FIG. 8 is a graph showing the results of repeating the procedure of: adding a catechin solution at 8.3 mL per minute for 5 minutes such that the amount of catechins added per minute was 0.1% (3 g of catechins/3000 mL), and the total amount of catechins added was 20% (600 g of catechins/3000 mL), at the concentration of tannase compared with the solution being 4 units; and carrying out a reaction for 10 minutes, wherein the content of EGCG in the reaction solution of catechins and tannase was measured every 30 minutes. Each numerical value (%) on the graph indicates the amount of catechins accumulated.

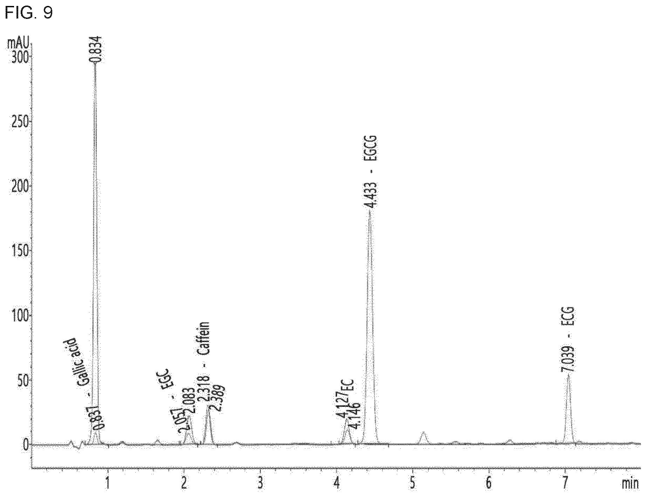

FIG. 9 is an HPLC chromatogram showing the analysis of the ingredients of the enzymatically treated green tea catechin products produced according to Comparative Example 1 and Example 1 of the present invention.

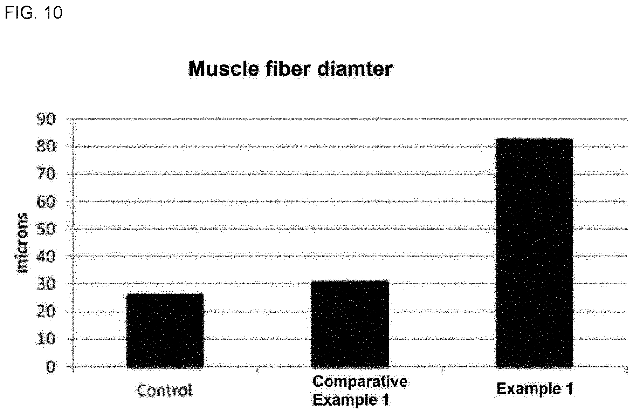

FIG. 10 is a graph showing the muscle fiber diameter of differentiated muscle cells, as a result of treating C2C12 precursor muscle cells with the enzymatically treated green tea catechin products produced according to Comparative Example 1 and Example 1 of the present invention.

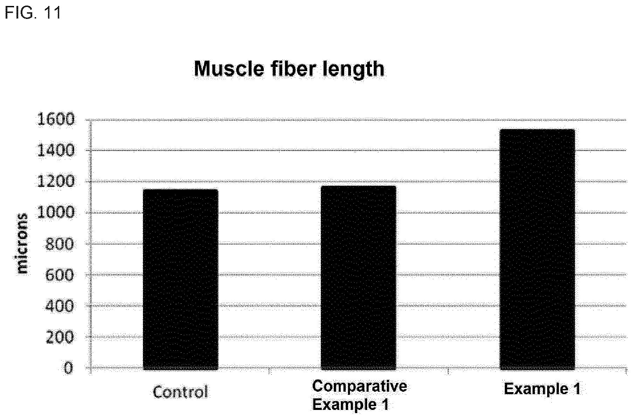

FIG. 11 is a graph showing the muscle fiber length of differentiated muscle cells, as a result of treating C2C12 precursor muscle cells with the enzymatically treated green tea catechin products produced according to Comparative Example 1 and Example 1 of the present invention.

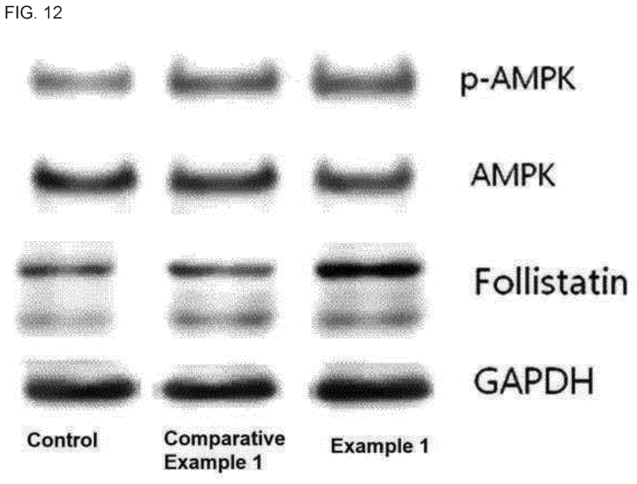

FIG. 12 is a Western blot image showing AMPK activity and muscle growth factor expression, as a result of treating C2C12 precursor muscle cells with the enzymatically treated green tea catechin products produced according to Comparative Example 1 and Example 1 of the present invention.

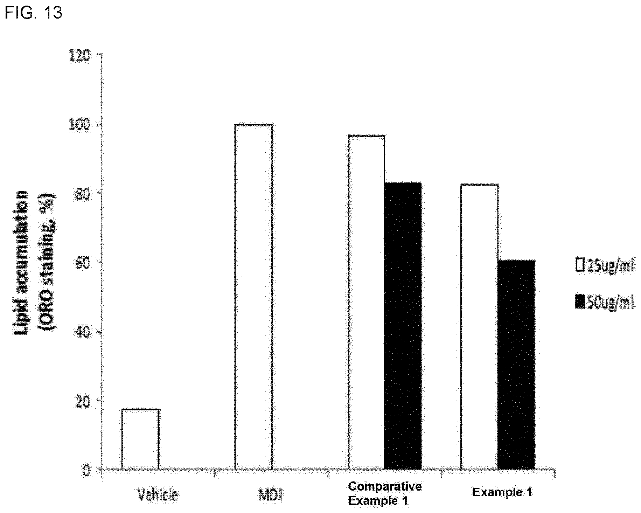

FIG. 13 is a graph showing lipid accumulation rate in adipose cells, as a result of treating 3T3-L1 precursor adipose cells with the enzymatically treated green tea catechin products produced according to Comparative Example 1 and Example 1 of the present invention at 25 $\mu$g/mL and 50 $\mu$g/mL each.

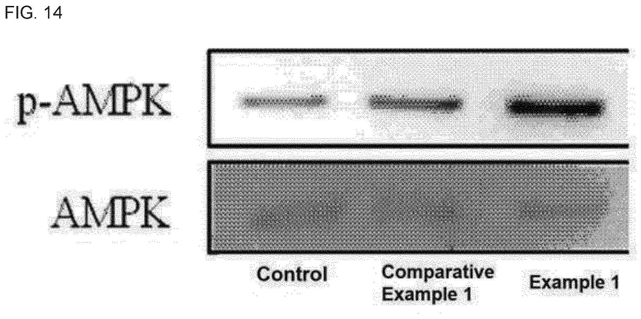

FIG. 14 is a Western blot image showing AMPK activity in adipose cells, as a result of treating 3T3-L1 precursor adipose cells with the enzymatically treated green tea catechin products produced according to Comparative Example 1 and Example 1 of the present invention.

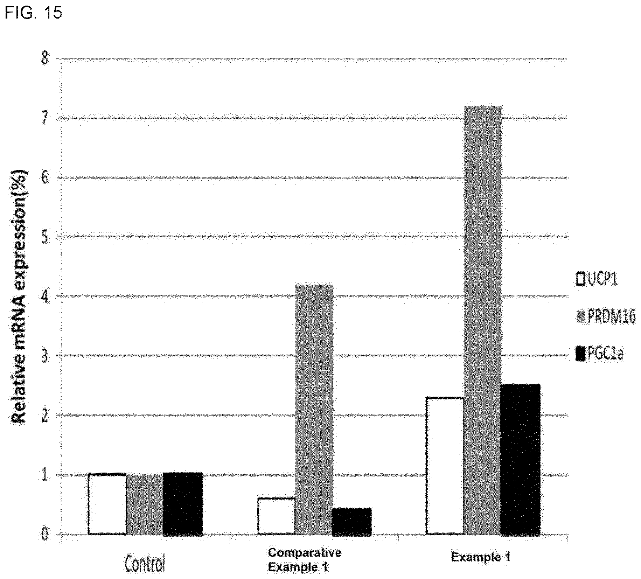

FIG. 15 is a graph showing mRNA expression of UCP1, PRDM16, and PGC1a, which are genes involved in the conversion of white adipose tissue into brown adipose tissue, as a result of treating 3T3-L1 precursor adipose cells with the enzymatically treated green tea catechin products produced according to Comparative Example 1 and Example 1 of the present invention.

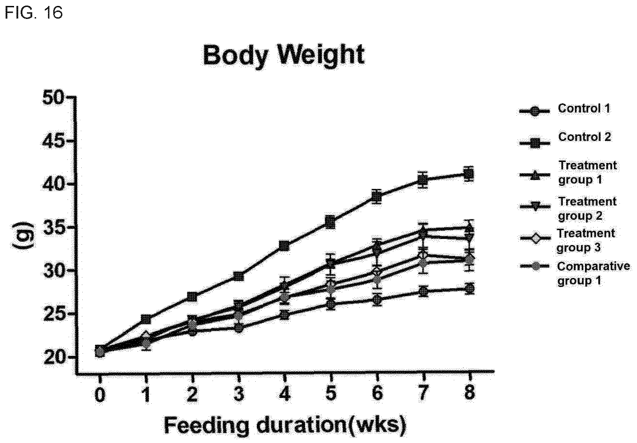

FIG. 16 is a graph showing body weight changes of mice treated with the enzymatically treated green tea catechin products produced according to Comparative Example 1 and Example 1 of the present invention. Control group 1 is a normal control group fed only a normal diet; Control group 2 is a negative control group fed only a high-fat diet; Treatment group 1 is a test group fed a high-fat diet and administered 50 mg/kg of the enzymatically treated catechin product produced according to Example 1; Treatment group 2 is a test group fed a high-fat diet and administered 100 mg/kg of the enzymatically treated catechin product produced according to Example 1; Treatment group 3 is a test group fed a high-fat diet and administered 200 mg/kg of the enzymatically treated catechin product produced according to Example 1; and Comparative group 1 is a test group fed a high-fat diet and administered 300 mg/kg of the enzymatically treated catechin product produced according to Comparative Example 1.

## Detailed Description of the Invention

In an accordance with an aspect of the present invention, there is provided a method for producing an enzymatically treated catechin product, the method including: a first step of preparing a tannase solution; a second step of adding a catechin solution to the tannase solution; a third step of reacting catechins and tannase contained in the solutions in the second step; and a fourth step of continuously repeating the second step and the third step.

In the present invention, by continuously repeating the step of adding a catechin solution to a tannase solution and the step of reacting catechins and tannase without the addition of a catechin solution, enzymatic reactions are completed in a short time without the inhibition of enzyme activity while the total amount of substrate usable in the reactions is increased, leading to mass-production of an enzymatically treated catechin product with reduced contents of EGCG and ECG and increased contents of gallic acid, EC, and EGC.

As used herein, the term "enzymatically treated catechin product" refers to a material obtained by reactions of catechins and an enzyme, and specifically may be one obtained by reactions of catechins and tannase as a gallate-degrading enzyme, but is not limited thereto. Such an enzymatically treated product may be used as it is, or may be used in a liquid or powder form by concentration under reduced pressure or freeze-drying.

As used herein, the term "catechin" refers to a kind of polyol which belongs to flavan-3-ols of the flavonoid group. Specifically, exemplary catechins are epigallocatechin gallate (EGCG), epicatechin gallate (ECG), epigallocatechin (EGC), epicatechin (EC), gallic acid, and the like.

As used herein, the term "catechin solution" refers to a liquid catechin, and may refer to a catechin dissolved in a solvent. Specifically, the solvent is not limited to the type thereof as long as it dissolves a catechin and the dissolved catechin can react with tannase therein, but specifically may be purified water.

The catechin used in the present invention may be not only a 100% pure catechin but also a mixture of the catechin with another material. Specifically, the content of the catechin in the mixture may be 1% to 99%, 1% to 80%, 1% to 75%, 20% to 99%, 20% to 80%, 20% to 75%, 30% to 75%, and more specifically 33% to 75%, but is not limited thereto.

The catechins in the present invention may be purchased from among commercially available catechins or may be obtained by extraction and/or fractionation from plants containing catechins.

In an aspect of the present invention, the catechin may be a green tea extract containing catechins or a fraction thereof, but is not limited thereto. In an exemplary embodiment of the present invention, an enzymatically treated catechin product can be produced using green tea catechins purchased from Anhui Redstar Pharmaceutical Corp. Ltd.

As used herein, the term "extract" refers to a resultant product, such as a liquid component obtained by immersing a desired material in various solvents and then conducting extraction at room temperature or in a warmed condition for a predetermined time, or a solid component obtained by removing the solvents from the liquid component. Furthermore, the term may be comprehensively interpreted to encompass, in addition to the resultant product, all of a diluted solution of the resultant product, a concentrate thereof, a crude or purified product thereof, a purified product thereof, and the like. A method of obtaining the extract is not particularly limited as long as green tea catechins can be obtained, and extraction may be conducted by a method commonly used in the art. Non-limiting examples of the extraction method may be hot-water extraction, ultrasonic extraction, filtration, reflux extraction, and the like, which may be conducted alone or in a combination of two or more thereof. Specifically, tea leaves and purified water may be placed in a low-temperature concentration extractor, followed by extraction.

The green tea extract in the present invention may be prepared into a fraction thereof before use.

As used herein, the term "fraction" refers to a resultant product obtained by conducting fractionation to separate a particular component or a group of particular components from a mixture containing various components.

A fractionation method of obtaining the fraction in the present invention is not particularly limited, and fractionation may be conducted by way of a method commonly used in the art. Examples thereof may include a solvent fractionation method performed by treatment with various solvents, an ultra-filtration fractionation method performed by passage through an ultra-filtration membrane with a predetermined molecular weight cut-off value, a chromatographic fractionation method performed by using various types of chromatography (manufactured for separation according to size, charge, hydrophobicity, or affinity), and a combination thereof. The kind of solvent used to obtain the fraction in the present invention is not particularly limited, and any solvent known in the art may be used. Non-limiting examples of the fractionation solvent may include water, an organic solvent, or a mixture solvent thereof, and examples of the organic solvent may include: a polar solvent, such as an alcohol having 1 to 4 carbon atoms, ethyl acetate, or acetone; a non-polar solvent, such as hexane or dichloromethane; or a mixture solvent thereof. Specifically, water, an alcohol having 1 to 4 carbon atoms, ethyl acetate, or a mixture solvent thereof may be used, and more specifically, ethanol or ethyl acetate may be used.

The content of catechins in the green tea extract or fraction may be 1% to 99%, 1% to 80%, 1% to 75%, 20% to 99%, 20% to 80%, 20% to 75%, 30% to 75%, and more specifically 33% to 75%, relative to the total weight of the extract or fraction, but is not limited thereto. In an exemplary embodiment of the present invention, green tea catechin solutions were prepared by dissolving 770 g of an ethanol fraction of a green tea extract having a catechin content of 34.5%, 430 g of an ethyl acetate fraction of a green tea extract having a catechin content of 62.1%, and 360 g of Chinese green tea catechins having a catechin content of 74.1% in 2 L of purified water, separately. Each of the green tea catechin additional solutions thus prepared was added at 16.6 mL per minute to a tannase solution for 5 minutes using a controlled-volume pump, followed by reaction for 10 minutes, and such procedure was repeated. After the addition of the substrate, sampling was conducted every 30 minutes to measure the content of EGCG, and as a result, it was identified that the enzyme reactions were all completed within 7 hours regardless of the content of catechins in the green tea extract.

As used herein, the term "tannase" refers to a tannin hydrolase, which is an enzyme that hydrolyzes an ester bond of tannin, methyl gallate, or the like. Specifically, the tannase in the present invention can degrade epicatechin gallate (ECG) into epicatechin (EC) and gallic acid and can degrade epigallocatechin gallate (EGCG) into epigallocatechin (EGC) and gallic acid. In an exemplary embodiment of the present invention, the kikoman enzyme was used, but is not limited thereto.

As used herein, the term "tannase solution" refers to a solution containing tannase, and which specifically may contain unreacted catechins or reaction products as well as tannase. In an exemplary embodiment of the present invention, the tannase solution may be a reaction solution after the second step.

The first step is a step of preparing a tannase solution. Specifically, the tannase solution may be prepared by adding tannase to purified water, followed by stirring, but is not limited thereto.

The concentration of tannase, in the first step of reacting the catechin solution and tannase, relative to the volume of the total solution, which is the sum of the volume of the tannase solution in the first step and the volume of the catechin solution added in the second step may be 1 U/mL to 50 U/mL, 1 U/mL to 45 U/mL, 1 U/mL to 40 U/mL, or 1 U/mL to 35 U/mL, specifically 1 U/mL to 20 U/mL, 1 U/mL to 16 U/mL, 1 U/mL to 10 U/mL, or 1 U/mL to 5 U/mL, and more specifically 4 U/mL, but is not limited thereto.

The volume of the total solution may be a final volume of the solution in the second solution after the second step is repeated once or more.

The first step may further include a step of activating tannase. Specifically, tannase and purified water may be placed in a reactor and activated by stirring at 100 rpm to 300 rpm for 5 minutes to 1 hour at 20°C to 60°C, and more specifically activated by stirring at 200 rpm for 30 minutes at 40°C, but is not limited thereto.

The first step may be performed at 20°C to 60°C, specifically 25°C to 60°C, 20°C to 55°C, 30°C to 60°C, 20°C to 50°C, 25°C to 55°C, 30°C to 50°C, and more specifically 35°C to 45°C, but is not limited thereto.

The first step may be performed at pH 3 to pH 6, specifically pH 3.5 to pH 6, 3 to pH 5.5, 4 to pH 6, 4 to pH 5, 3.5 to pH 5.5, 4 to pH 5.5, 3.5 to pH 5, and more specifically in the range of pH 4.5 to pH 5.5, but is not limited thereto.

The first step may be performed by stirring at a rate of 0.1 rpm to 1000 rpm, specifically 10 rpm to 1000 rpm, 0.1 rpm to 900 rpm, 100 rpm to 900 rpm, 150 rpm to 900 rpm, 100 rpm to 800 rpm, 100 rpm to 700 rpm, 100 rpm to 600 rpm, 100 rpm to 500 rpm, 100 rpm to 400 rpm, and more specifically 100 rpm to 300 rpm, but is not limited thereto.

The first step may be performed for 1 to 60 minutes, specifically 4 to 60 minutes, 1 to 40 minutes, 4 to 40 minutes, 1 to 20 minutes, and more specifically 5 to 20 minutes, but is not limited thereto.

The second step is a step of adding a catechin solution to the tannase solution.

The tannase solution in the second step may be a solution containing tannase, and specifically may contain unreacted catechins or a reaction product as well as tannase. In an exemplary embodiment of the present invention, the tannase solution may be a reaction solution after the second step.

The amount of catechins added per minute in the second step, relative to the volume of the total solution, which is the sum of the volume of the tannase solution in the first step and the volume of the catechin solution added in the second step, may be 0.01% (w/v) to 20% (w/v), specifically 0.01% (w/v) to 15% (w/v), 0.01% (w/v) to 10%(w/v), 0.01% (w/v) to 5% (w/v), and 0.01% (w/v) to 1% (w/v), and more specifically 0.01% (w/v) to 0.8% (w/v), 0.01% (w/v) to 0.6% (w/v), 0.01% (w/v) to 0.4% (w/v), 0.01% (w/v) to 0.2% (w/v), and still more specifically 0.1% (w/v), but is not limited thereto.

The second step may be performed for 1 to 60 minutes, specifically 4 to 60 minutes, 1 to 40 minutes, 4 to 40 minutes, 1 to 20 minutes, and more specifically 5 to 20 minutes, but is not limited thereto.

The total amount of the catechins added in the second step, relative to the volume of the total solution, which is the sum of the volume of the tannase solution in the first step and the volume of the catechin solution added in the second step, may be 0.01% (w/v) to 40% (w/v), specifically 0.01% (w/v) to 35% (w/v), 0.01% (w/v) to 30% (w/v), 0.01% (w/v) to 25% (w/v), 0.01% (w/v) to 20% (w/v), 0.01% (w/v) to 15% (w/v), 0.01% (w/v) to 10% (w/v), 0.3% (w/v) to 40% (w/v), 0.3% (w/v) to 35% (w/v), 0.3% (w/v) to 30% (w/v), 0.3% (w/v) to 25% (w/v), 0.3% (w/v) to 20% (w/v), 0.3% (w/v) to 15% (w/v), 0.3% (w/v) to 10% (w/v), more specifically 0.01% (w/v) to 2% (w/v), 0.01% (w/v) to 1.5% (w/v), 0.3% (w/v) to 2% (w/v), 0.3% (w/v) to 1.5% (w/v), 0.4% (w/v) to 1.1% (w/v), and still more specifically 0.5% (w/v), but is not limited thereto.

The volume of the total solution may be a final volume of the solution in the second solution after the second step is repeated once or more.

The third step is a step of reacting catechins and tannase contained in the solutions in the second step without the addition of the catechin solution.

The third step may be performed for 1 to 60 minutes. The third step may be performed for specifically 4 to 60 minutes, 1 to 40 minutes, 4 to 40 minutes, 1 to 20 minutes, 1 to 11 minutes, 4 to 11 minutes, and more specifically 10 minutes, but is not limited thereto.

The reaction temperature, pH, and stirring rate in the third step are identical to the reaction temperature, pH, and stirring rate in the first step.

The fourth step is a step of continuously repeating the second step and the third step.

The fourth step of continuously repeating the second step and the third step may be performed until the amount of catechins added in the second step, relative to the volume of the total solution, which is the sum of the volume of the tannase solution in the first step and the volume of the catechin solution added in the second step, is 1% (w/v) to 90% (w/v), specifically 5% (w/v) to 90% (w/v), 10% (w/v) to 90% (w/v), 1% (w/v) to 21% (w/v), 1% (w/v) to 40% (w/v), 1% (w/v) to 60% (w/v), 5% (w/v) to 21% (w/v), 5% (w/v) to 40% (w/v), 5% (w/v) to 60% (w/v), 10% (w/v) to 40% (w/v), 10% (w/v) to 60% (w/v), 10% (w/v) to 21% (w/v), and more specifically 12% (w/v), but is not limited thereto.

In an exemplary embodiment of the present invention, 2000 g of purified water was placed in a reactor, and citric acid and sodium bicarbonate were added to acid and alkali pumps of a fermenter, respectively, and thereafter, the pH was set to 5.0, which is an appropriate pH for enzyme treatment. To the purified water, 24 g of tannase (Tannase KTFH, 500 U/g) was added, and activated by stirring at 200 rpm for 30 minutes at 40°C (first step). To add green tea catechins to the reactor, 360 g of green tea catechins was dissolved in purified water to prepare 1 L of a green tea catechin solution. The green tea catechin solution thus prepared was added to the tannase solution at a rate of 8.3 mL per minute through a controlled-volume pump for 5 minutes (second step). That is, in the second step, the green tea catechins were added at 3 g per minute, and hence 0.1% (=

$$\frac{3 \text{ g of green catechins}}{2000 \text{ mL of tannase solution} + 1000 \text{ mL of catechin solution}} \times 100$$

) per minute of catechins were added, and the total amount of catechins added for 5 minutes in the second step was 0.5% (=

$$\frac{15 \text{ g of green catechins}}{2000 \text{ mL of tannase solution} + 1000 \text{ mL of catechin solution}} \times 100$$

).

After the addition of the green tea catechin solution, green tea catechins and tannase contained in the solutions in the second step were allowed to react with each other for 10 minutes (third step). Then, a green tea catechin solution was added, at a rate at which 3 g per minute of green tea catechins (0.1 % of green tea catechins) was added, to the reaction solution of green tea catechins and tannase, for 5 minutes, followed by reaction for 10 minutes, and such procedure was repeated. The second step and the third step were repeated until the total amount of green tea catechins added was 360 g

(12% (=

(

$$\frac{360 \text{ g of green catechins}}{2000 \text{ mL of tannase solution} + 1000 \text{ mL of catechin solution}} \times 100$$

)

of green tea catechins) (fourth step). To investigate the enzyme inactivation inhibitory effect by the further addition of the substrate, the procedure of sampling the reaction solution every 15 minutes to measure the content of EGCG was repeated for 6 hours to observe the change over time. Even after the further addition of catechins was completed, the change in EGCG content was observed for 30 minutes. As a result, it was identified that the enzyme reactions were completed within 6 hours and 30 minutes and the enzyme was not inactivated even after the addition of green tea catechins was completed (FIG. 6).

In another exemplary embodiment of the present invention, 2000 g of purified water was placed in a reactor, and citric acid and sodium bicarbonate were added to acid and alkali pumps of a fermenter, respectively, and thereafter, the pH was set to 5.0, which is an appropriate pH for enzyme treatment. Thereafter, 24 g of tannase (Tannase KTFH, 500 U/g) was added to the reactor and activated by stirring at 200 rpm for 30 minutes at 40°C. To add green tea catechins to the reactor, 360 g of green tea catechins was dissolved in purified water to prepare 1 L of a green tea catechin solution. The green tea catechin solution thus prepared was added to the tannase solution at a rate of 8.3 mL per minute through a controlled-volume pump for 10 minutes (second step). That is, in the second step, the green tea catechins were added at       3       g       per       minute,       and       hence,       0.1%       (=

$$\frac{3 \text{ g of green catechins}}{2000 \text{ mL of tannase solution} + 1000 \text{ mL of catechin solution}} \times 100$$

) per minute of catechins were added, and the total amount of catechins added for 10 minutes in the second step was 1% (=

$$\frac{30 \text{ g of green catechins}}{2000 \text{ mL of tannase solution} + 1000 \text{ mL of catechin solution}} \times 100$$

).

After the addition of the green tea catechin solution, green tea catechins and tannase contained in the solutions in the second step were allowed to react with each other for 5 minutes (third step). Then, a green tea catechin solution was added, at a rate at which 3 g per minute of green tea catechins (0.1 % of green tea catechins) was added, to the reaction solution of green tea catechins and tannase for 10 minutes, followed by reaction for 5 minutes, and such procedure was repeated. The second step and the third step were repeated until the total amount of green tea catechins added       was       360       g       (12%       (=       (

$$\frac{360 \text{ g of green catechins}}{2000 \text{ mL of tannase solution} + 1000 \text{ mL of catechin solution}} \times 100$$

) of green tea catechins) (fourth step). To investigate the enzyme inactivation inhibitory effect by the further addition of the substrate, the procedure of sampling the reaction solution every 15 minutes to measure the content of EGCG was repeated for 3 hours to observe the change thereof over time. Even after the further addition of catechins was completed, the change in EGCG content was observed for 3 hours. As a result, it was identified that the enzyme reactions were completed within 6 hours and the enzyme was not inactivated even after the addition of green tea catechins was completed (FIG. 7).

The method for producing an enzymatically treated catechin product may further include: inactivating tannase; and/or filtering, concentrating, and/or drying the reaction solution.

The inactivating of tannase may be performed by using various methods known in the art, specifically, thermal treatment, but is not limited thereto. In an embodiment, the method for producing an enzymatically treated catechin product may further include, after the fourth step, inactivating tannase by subjecting the reaction solution to thermal treatment at 80°C to 120°C for 5 minutes to 4 hours. Specifically, tannase may be inactivated by subjecting the reaction solution to thermal treatment at 90°C to 100°C for 10 to 30 minutes, but is not limited thereto.

In addition, the filtering, concentrating, and/or drying of the reaction solution in each step may be conducted by using various methods known in the art. In an embodiment, the method for producing an enzymatically treated catechin product may further include: filtering, concentrating, and/or drying the reaction solution completed the fourth step. In the method, the filtering may be conducted using a filter paper or a vacuum filter, but is not limited thereto, and the concentrating may specifically be conducted using a rotary evaporation concentrator, but is not limited thereto. The drying may be conducted by drying under reduced pressure, vacuum drying, boiling drying, spray drying, or freeze-drying, but is not limited thereto. More specifically, in an exemplary embodiment of the present invention, the reaction solution completed the reaction in the fourth step was subjected to thermal treatment at 80°C for 30 minutes to inactivate the enzyme, and then the reaction solution was freeze-dried.

The enzymatically treated catechin product produced by way of the method for producing an enzymatically treated catechin product may be an enzymatically treated catechin product containing less than 1 part by weight of the sum of epigallocatechin gallate (EGCG) and epicatechin gallate (ECG) relative to 100 parts by weight of the sum of gallic acid, epigallocatechin gallate (EGCG), epicatechin gallate (ECG), epigallocatechin (EGC), and epicatechin (EC).

In accordance with another aspect of the present invention, there is provided an enzymatically treated catechin product produced by way of the method for producing an enzymatically treated catechin product, the enzymatically treated catechin product containing less than 1 part by weight of the sum of epigallocatechin gallate (EGCG) and epicatechin gallate (ECG) relative to 100 parts by weight of the sum of gallic acid, epigallocatechin gallate (EGCG), epicatechin gallate (ECG), epigallocatechin (EGC), and epicatechin (EC).

Specifically, the enzymatically treated catechin product produced by way of the method for producing an enzymatically treated catechin product may contain less than 0.9 parts by weight, less than 0.8 parts by weight, less than 0.7 parts by weight, less than 0.6 parts by weight, less than 0.5 parts by weight, less than 0.4 parts by weight, less than 0.3 parts by weight, less than 0.2 parts by weight, or less than 0.1 parts by weight of the sum of epigallocatechin gallate (EGCG) and epicatechin gallate (ECG) relative to 100 parts by weight of the sum of gallic acid, epigallocatechin gallate (EGCG), epicatechin gallate (ECG), epigallocatechin (EGC), and epicatechin (EC), but is not limited thereto. In an exemplary embodiment of the present invention, the contents of epigallocatechin gallate (EGCG) and epicatechin gallate (ECG) were not measured in the enzymatically treated catechin product produced by way of the method for producing an enzymatically treated catechin product (Table 4).

As such, the enzymatically treated catechin product produced by way of the method for producing an enzymatically treated catechin product exhibits effects of having reduced contents of epigallocatechin gallate (EGCG) and epicatechin gallate (ECG), which cause bitter and astringent tastes and inhibit digestive enzymes, and increased contents of gallic acid, epicatechin (EC), and epigallocatechin (EGC).

In accordance with another aspect of the present invention, there is provided a pharmaceutical composition for preventing or treating obesity or sarcopenia, the composition containing the enzymatically treated catechin product.

In accordance with another aspect of the present invention, there is provided use of the enzymatically treated catechin product for preventing or treating obesity or sarcopenia.

The "enzymatically treated catechin product" is as described above.

As used herein, the term "obesity" refers to a condition or disease that has excessive body fat caused by energy imbalance.

In an exemplary embodiment of the present invention, as a result of culturing 3T3-L1 precursor adipose cells by adding an enzymatically treated green tea catechin product, the effects of inhibiting the differentiation of precursor adipose cells into adipose cells and the generation of triglycerides and the AMPK activation effect were exhibited, and it was therefore identified that the enzymatically treated green tea catechin product can be used as a pharmaceutical composition for preventing or treating obesity (FIGS. 13 and 14).

In another exemplary embodiment of the present invention, as a result of culturing 3T3-L1 precursor adipose cells by adding an enzymatically treated green tea catechin product, the effect of increasing the expression of UCP1 and PRDM16, which cause the conversion of white adipose tissue into brown adipose tissue, was exhibited, and it was therefore identified that the enzymatically treated green tea catechin product can be used as a pharmaceutical composition for preventing or treating obesity (FIG. 15).

In still another exemplary embodiment of the present invention, as a result of oral administration of an enzymatically treated green tea catechin product to mice fed a high-fat diet, the level of the body weight gain was similar to that of a control group fed a normal diet, and it was therefore identified that the enzymatically treated green tea catechin product can be used as a pharmaceutical composition for preventing or treating obesity (FIG. 16).

As used herein, the term "sarcopenia" refers to the gradual weakness of density and functions of muscles, which is known to be caused by progressive degeneration and destruction of motor neurons or muscle cells in the spinal nerves or diencephalon.

In an exemplary embodiment of the present invention, as a result of culturing C2C12 muscle cells by adding an enzymatically treated green tea catechin product, the effects of promoting muscle cell differentiation, activating p-AMPK, and increasing follistatin expression were exhibited, and it was therefore identified that the enzymatically treated green tea catechin product can be used as a pharmaceutical composition for preventing or treating sarcopenia (FIGS. 10 to 12).

As used herein, the term "prevention" refers to all actions that inhibit or delay obesity or sarcopenia through the administration of the pharmaceutical composition of the present invention, and the term "treatment" refers to all actions that alleviate or favorably change obesity or sarcopenia by administration of the pharmaceutical composition of the present invention.

As used herein, the term "pharmaceutical composition" refers to one prepared for the purpose of preventing or treating a disease, wherein the pharmaceutical composition may be formulated in various forms according to typical methods. For example, the pharmaceutical composition may be formulated into oral formulations, such as a powder, granules, a tablet, a capsule, a suspension, an emulsion, and a syrup, or may be formulated in the forms of an externally applied preparation and a sterile injectable solution.

The pharmaceutical composition of the present invention may be prepared in the form of a pharmaceutical composition for preventing or treating obesity or sarcopenia, the composition further containing an appropriate carrier, excipient, or diluent that is commonly used in the preparation of a pharmaceutical composition, wherein the carrier may include a carrier which does not occur naturally. Specifically, the pharmaceutical composition may be formulated in the form of: an oral formulation, such as a powder, granules, a tablet, a capsule, a suspension, an emulsion, a syrup, or an aerosol; an externally applied preparation; a suppository; and a sterile injectable solution, according to commonly used methods, respectively. In the present invention, examples of the carrier, excipient, and diluent that may be contained in the pharmaceutical composition may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and a mineral oil. Specifically, the pharmaceutical composition, when made into a preparation, may be prepared using a diluent or an excipient, such as a filler, an extender, a binder, a wetting agent, a disintegrant, or a surfactant that is usually used. Solid preparations for oral administration include a tablet, a pill, a powder, granules, a capsule, and the like, and such solid preparations may be prepared by mixing with at least one excipient, for example, starch, calcium carbonate, sucrose or lactose, gelatin, or the like. Alternatively, lubricants, such as magnesium stearate and talc, may also be used in addition to simple excipients. Liquid preparation for oral administration correspond to a suspension, a liquid for internal use, an emulsion, a syrup, or the like, and examples thereof may include not only simple diluents that are frequently used, such as water and liquid paraffin, but also several types of excipients, such as a wetting agent, a sweetener, a flavoring agent, and a preservative. Examples of preparations for parenteral administration include a sterilized aqueous solution, a non-aqueous solvent, a suspension, an emulsion, a freeze-dried preparation, and a suppository. As a non-aqueous solvent and suspension, propylene glycol, polyethylene glycol, a vegetable oil such as olive oil, an injectable ester such as ethyl oleate, and the like may be used. As a substrate for the suppository, Witepsol, macrogol, Twin 61, cacao butter, laurin butter, glycerogelatin, or the like may be used.

The specific dose of the pharmaceutical composition of the present invention may be variously selected by a person skilled in the art depending on factors such as formulation method, condition and body weight of a patient, gender and age of a patient, severity of a disease, dosage form of a drug, administration route and period, excretion speed, and response sensitivity, and the dose and the number of times of dosing are not intended to limit the scope of the present invention in any way.

The pharmaceutical composition of the present invention may be administered to mammals, such as rats, mice, livestock, and humans, via various routes. All manners of administration may be predicted, and the pharmaceutical composition may be administered according to a desired purpose via a route of administration, such as eyedrop administration, intraperitoneal administration, intravenous administration, intramuscular administration, subcutaneous administration, intradermal administration, transdermal patch administration, oral administration, intranasal administration, intrapulmonary administration, and rectal administration.

In accordance with another aspect of the present invention, there is provided a food composition for preventing or alleviating obesity or sarcopenia, the composition containing the enzymatically treated catechin product.

As used herein, the terms "enzymatically treated catechin product", "obesity", "sarcopenia", and "prevention" are as described above.

As used herein, the term "alleviation" refers to all actions that favorably change obesity or sarcopenia through the administration of the composition.

As used herein, the term "food" includes meats, sausages, breads, chocolates, candies, snacks, confectionaries, pizzas, ramens, other noodles, gums, dairy products including ice creams, various kinds of soups, beverages, teas, drinks, alcoholic beverages, vitamin complexes, functional foods, health foods, and the like, and may encompass all foods in the ordinary meaning thereof.

The functional food, being the same term as food for special health use (FoSHU), refers to a food with high medicinal and medical effects to efficiently exhibit a bio-regulatory function in addition to a function of nutrient supply. The term "functional" refers to controlling nutrients for the structure or functions of the human body or providing beneficial effects to health purposes, such as physiological effects. The food of the present invention may be manufactured by way of a method that is commonly used in the art, and during the manufacturing, the food may be manufactured by adding raw materials and ingredients that are commonly added in the art. In addition, the food may be manufactured in any formulation without limitation as long as it is acceptable as a food. The food composition of the present invention may be prepared in various formulations, and unlike general medicines, the food composition has an advantage in that there is no side effect that may occur when a drug is taken for a long time, because of using the food as a raw material, and has excellent portability, so that the food of the present invention can be ingested as a supplement for enhancing immune-boosting effects.

The health food refers to a food that has an effect of actively maintaining or improving health, as compared with general foods, and the health supplement food refers to a food to be used for health supplement. In some cases, the terms of health functional food, health food, and health supplement food are used interchangeably with one another.

Specifically, the functional food may be a food manufactured by adding an enzymatically treated catechin product to food materials, such as beverages, teas, flavors, gum, and snacks, or manufactured as a capsule, a powder, a suspension, or the like. The ingestion of such a functional food means bringing about a particular effect on health, but unlike other common medicines, the functional food has an advantage of avoiding side effects associated with long-term administration of drugs, because of using the food as a raw material.

The food composition of the present invention can be ingested as usual, and thus is expected to have high immune-boosting effects, and therefore, the food composition is very useful.

The composition may further contain a physiologically acceptable carrier, wherein the kind of carrier is not particularly limited, and any carrier can be used as long as it is a carrier commonly used in the art.

The composition may further contain additional ingredients that are commonly used in food compositions to enhance the smell, taste, visual appearance, and the like. For example, the composition may contain vitamins A, C, D, E, B1, B2, B6, or B12, niacin, biotin, folate, pantothenic acid, and the like. The composition may also contain minerals, such as zinc (Zn), iron (Fe), calcium (Ca), chromium (Cr), magnesium (Mg), manganese (Mn), copper (Cu), and chromium (Cr). The composition may also contain amino acids, such as lysine, tryptophan, cysteine, and valine.

The composition may also contain antiseptics (potassium sorbate, sodium benzoate, salicylic acid, sodium dehydroacetate, *etc.),* disinfecting agents (bleaching powder and high-test bleaching powder, sodium hypochlorite, *etc.),* antioxidants (butylhydroxyanisole (BHA), butylhydroxytoluene (BHT), *etc.),* colorants (tar dye, etc.), color fixing agents (sodium nitrate, sodium nitrite, *etc.),* bleaching agents (sodium sulfite), seasoning agents (monosodium glutamate, *etc.),* sweeteners (dulcin, cyclamate, saccharine, sodium, *etc.),* flavoring agents (vanillin, lactones, *etc.),* blowing agents (alum, potassium hydrogen tartrate, *etc.),* fortifying agents, emulsifying agents, thickening agents, coating agents, gum bases, anti-foaming agents, solvents, modifiers, and the like. The additives may be selected according to food type, and may be used in suitable amounts.

The enzymatically treated catechin product may be added as it is, or may be used with other foods or food ingredients, and may be appropriately used according to typical methods. The amount of the active ingredient mixed may be appropriately determined according to the purpose of use (prevention, health, or therapeutic treatment) thereof. However, when consumed for a long period of time for health and sanitary purposes, the active ingredient may be contained in a content below the range, and due to no safety problems, may be used in an amount above the range.

The food composition of the present invention may be used as, for example, a health beverage composition. In such case, the health beverage composition, like common beverages, may contain additional ingredients, such as various flavoring agents and natural carbohydrates. The above-described natural carbohydrates may be: monosaccharides such as glucose and fructose; disaccharides such as maltose and sucrose; polysaccharides such as dextrin and cyclodextrin; and sugar alcohols such as xylitol, sorbitol, and erythritol. As sweeteners, natural sweetens, such as thaumatin and a stevia extract, and synthetic sweeteners, such as saccharin and aspartame, may be used.

Furthermore, the health beverage composition may further contain various nutritional supplements, vitamins, electrolytes, flavorings, coloring agents, pectic acid and salts thereof, alginic acid and salts thereof, organic acids, protective-colloidal thickeners, pH regulators, stabilizing agents, preservatives, glycerin, alcohols or carbonating agents, or the like. Furthermore, the health beverage composition may contain fruit flesh for manufacturing natural fruit juices, fruit juice drinks, or vegetable drinks. These ingredients may be used independently or in a mixture thereof.

The food composition of the present invention may contain the enzymatically treated catechin product in various weight percentages as long as it can exhibit an effect of preventing or alleviating obesity or sarcopenia, but specifically, the food composition may contain the enzymatically treated catechin product in 0.00001 wt% to 100 wt% or 0.01 wt% to 80 wt% relative to the total weight of the food composition.

In accordance with another aspect of the present invention, there is provided a method for preventing or treating obesity or sarcopenia in a subject in need thereof, the method including administering to the subject the enzymatically treated catechin product.

As used herein, the terms "enzymatically treated catechin product", "obesity", "sarcopenia", "prevention", "treatment", and "administration" are as described above.

As used herein, the term "subject" may refer to all animals including humans which have developed or are likely to develop obesity or sarcopenia. The animals may be mammals including not only humans but also cattle, horses, sheep, pigs, goats, camels, antelopes, dogs, cats, and the like in need of treating symptoms similar thereto, but are not limited thereto.

Specifically, the prevention or treatment method of the present invention may include a step of conducting single or multiple administration of the composition in a pharmaceutically effective amount to a subject that has developed or is likely to develop obesity or sarcopenia.

## Mode for Carrying Out the Invention

Hereinafter, constitutions and effects of the present invention will be described in detail with reference to exemplary embodiments. However, these exemplary embodiments are used for illustration only, and the scope of the present invention is not limited to these exemplary embodiments.

The catechins used in the present invention are green tea catechins, and purchased from Anhui Redstar Pharmaceutical Corp. Ltd., and for tannase, which is a green tea catechin-degrading enzyme, 500 U/g Tannase-KTFH was purchased from Kikkoman.

## Comparative Example 1: Production of enzymatically treated green tea catechin product without further addition of catechin

To a reactor, 360 g of green tea catechins and 3000 g of purified water were added, and the pH was adjusted to 5.0, which corresponds to an appropriate pH for enzymatic treatment, while adding citric acid and sodium bicarbonate, and then stirring was conducted at 60 rpm for 5 minutes. After 24 g of tannase was added to the reaction solution, the reaction was conducted at 40°C for 6 hours, and then the enzyme was inactivated by reaction at 80°C for 30 minutes, thereby producing an enzymatically treated product.

## Comparative Example 2: Production of enzymatically treated green tea catechin product and measurement of EGCG content

## Comparative Example 2-1: When total amount of catechins added was 12% by continuously adding catechin powder by 1%

The pH was adjusted to 5.0, which corresponds to an appropriate pH for enzymatic treatment, by adding citric acid and sodium bicarbonate to 500 g of purified water, and thereafter, 4 g of tannase was added, and then activated by stirring at 60 rpm at 40°C for 5 minutes. After 5 g of green tea catechins were added to the tannase solution, the reaction was conducted at 40°C, and then sampling was conducted every 30 minutes to measure the EGCG content.

As a result, as shown in FIG. 3, at an enzyme concentration of 4 U/mL, 1% of catechins were completely degraded within 30 minutes, and thus 9% of catechins were completely degraded within 4 hours and 30 minutes. From the concentration of catechins compared with the solution being 10%, it took 1 hour to degrade 1% of catechins, and thus a total of 12% of catechins were completely degraded for 7 hours and 30 minutes.

**Comparative Example 2-2: When total amount of catechins added was 12% (360 g of catechins/3000 mL) by continuously adding catechin solution at 8.3 mL per minute such that amount of catechins added per minute was 0.1% (3 g of catechins/3000 mL)**

After 2000 g of purified water was placed in a reactor, citric acid and sodium bicarbonate were added to acid and alkali pumps of a fermenter, respectively, and then the pH was set to 5.0, which corresponds to an appropriate pH for enzyme treatment. After 24 g of tannase was added to the purified water, the tannase was activated by stirring at 200 rpm for 30 minutes at 40°C. For addition of green tea catechins to the reactor, 360 g of green tea catechins was dissolved in purified water to prepare 1 L of a green tea catechin solution. The green tea catechin solution thus prepared was continuously added at a rate at which catechins were added at 3 g (0.1% of the substrate) per minute through a controlled-volume pump for 2 hours, and the reaction solution was sampled every 15 minutes to measure the EGCG content. Even after catechin addition was ended, the change in EGCG content was observed for 3 hours.

As a result, as shown in FIG. 4, the EGCG content did not increase by 45 minutes after the start of catechin addition, and EGCG started to accumulate after 1 hour, and the enzyme was completely inactivated after 2 hours, at which time catechin addition was ended (12% of the substrate). Based on the results that the amount of EGCG accumulated compared with the amount of substrate added remained the same from 1 hour and 30 minutes, the substrate inhibition threshold indicating the complete inhibition of the enzyme was defined between 4 mg/mL and 8 mg/mL in terms of EGCG content under the above test conditions, and the continuous addition of the substrate thereafter was determined as resulting in the complete inactivation of the enzyme.

**Comparative Example 2-3: When total amount of catechins added was 12% (360 g of catechins/3000 mL) by continuously adding catechin solution at 6 mL per minute such that amount of catechins added per minute was 0.07% (2 g of catechins/3000 mL)**

After 2000 g of purified water was placed in a reactor, citric acid and sodium bicarbonate were added to acid and alkali pumps of a fermenter, respectively, and then the pH was set to 5.0, which corresponds to an appropriate pH for enzyme treatment. After 24 g of tannase was added to the purified water, the tannase was activated by stirring at 200 rpm for 30 minutes at 40°C. For addition of green tea catechins to the reactor, 360 g of green tea catechins was dissolved in purified water to prepare 1 L of a green tea catechin solution. The green tea catechin solution thus prepared was continuously added at a rate at which catechins were added at 2 g (0.07% of the substrate) per minute through a controlled-volume pump for 3 hours, and the reaction solution was sampled every 15 minutes to measure the EGCG content. Even after catechin addition was ended, the change in EGCG content was observed for 3 hours.

As shown in FIG. 5, EGCG started to accumulate 1 hour after the start of substrate addition, and the EGCG content steadily increased by 3 hours (12%), at which time the substrate addition was ended. However, the enzyme was not inactivated even after the ending of substrate addition, and thus the enzymatic treatment was completed at five hours. It was identified through the test results that when the concentration of the substrate did not exceed the irreversible enzyme inactivation concentration even though the concentration of EGCG reached the substrate inhibition threshold to result in the accumulation EGCG, the enzyme activity can be maintained, if sufficient time is given, thereby enabling the treatment of the accumulated substrates. However, it was identified that the EGCG content at the ending of substrate addition was 7 mg/mL, which did not belong to the irreversible enzyme inactivation section but was included in the substrate inhibition threshold, causing a risk of enzyme inactivation in the mass production.

**Example 1: Production of enzymatically treated green tea catechin product**

After 2000 g of purified water was placed in a reactor, citric acid and sodium bicarbonate were added to acid and alkali pumps of a fermenter, respectively, and then the pH was set to 5.0, which corresponds to an appropriate pH for enzyme treatment. After 24 g of tannase was added to the reaction solution, the tannase was activated by stirring at 200 rpm for 30 minutes at 40°C. For addition of green tea catechins to the reactor, 360 g of green tea catechins was dissolved in purified water to prepare 1 L of a green tea catechin solution. The green tea catechin solution thus prepared was added at a rate at which catechins were added at 3 g (0.1 % of the substrate) per minute, through a controlled-volume pump for 5 minutes, followed by reaction for 10 minutes, and such procedure was repeated to carry out the reaction for 6 hours. The reaction solution was reacted at 80°C for 30 minutes to inactivate the enzyme, and then the reaction solution was freeze-dried.

**Example 2: Production of enzymatically treated green tea catechin product and measurement of EGCG content**

The catechins used in the present invention were green tea catechins, and purchased from Anhui Redstar Pharmaceutical Corp. Ltd., and for tannase, which is a green tea catechin-degrading enzyme, 500 U/g of Tannase-KTFH was purchased from Kikkoman.

Examples 2-1 to 2-3 were carried out under conditions shown in Table 1 below.

TABLE 1

| Step | Specification | Example 2-1 | Example 2-2 | Example 2-3 |
|---|---|---|---|---|
| First step of preparing tannase solution | Purified water (g) | 2,000 | 2,000 | 1,400 |
| | Tannase (g) | 24 | 24 | 24 |
| Second step of adding catechin solution to tannase solution | Concentration of catechin solution added (g/L) | 360 | 360 | 375 |
| | Amount of catechins added per minute (g) | 3 | 3 | 3 |
| | Amount of catechins added per minute (% (w/v)) | 0.1 | 0.1 | 0.1 |
| | Time taken in second step (min) | 5 | 10 | 5 |
| | Total amount of catechins added in second step (% (w/v)) | 0.5 | 1 | 0.5 |
| Step of reacting catechins and tannase | Time taken in third step (min) | 10 | 5 | 10 |
| Fourth step of continuously repeating second step and third step | Number of times of catechin addition (times) | 24 | 12 | 40 |
| | Time taken in second to fourth steps (hours) | 6 | 3 | 10 |
| | Total amount of substrate added (% (w/v)) | 12 | 12 | 20 |

**Example 2-1: When total amount of catechins added was 12% (360 g of catechins/3000 mL) due to addition of catechin solution at 8.3 mL per minute for 5 minutes such that amount of catechins added per minute was 0.1% (3 g of catechins/3000 mL) and subsequent reaction for 5 minutes**

After 2000 g of purified water was placed in a reactor, citric acid and sodium bicarbonate were added to acid and alkali pumps of a fermenter, respectively, and then the pH was set to 5.0, which corresponds to an appropriate pH for enzyme treatment. After 24 g of tannase was added to the purified water, the tannase was activated by stirring at 200 rpm for 30 minutes at 40°C. For addition of green tea catechins to the reactor, 360 g of green tea catechins was dissolved in purified water to prepare 1 L of a green tea catechin solution. The green tea catechin solution thus prepared was added at a rate at which catechin was added at 3 g per minute (0.1% of the substrate) through a controlled-volume pump for 5 minutes, followed by reaction for 10 minutes, and such procedure was repeated. To investigate the enzyme inactivation inhibitory effect by the further addition of the substrate, the procedure of sampling the reaction solution every 15 minutes to measure the EGCG content was repeated for 6 hours to observe the change thereof over time. Even after catechin addition was completed, the change in EGCG content was observed for 30 minutes.

As a result, as shown in FIG. 6, EGCG started to accumulate 3 hours and 15 minutes after the start of green tea catechin addition, and the EGCG content steadily increased by 6 hours (12%), at which time the green tea catechin addition was ended. However, the EGCG content converged to zero 30 minutes after the ending of green tea catechin addition, and thus the degradation reaction was completed, and it was identified that the enzyme was not inactivated even after the ending of green tea catechin addition. It was also identified that the maximum content of EGCG accumulated was 1 mg/mL, which was lower than 4 mg/mL to 8 mg/mL, corresponding to the substrate inhibition threshold.

**Example 2-2: When total amount of catechins added was 12% (360 g of catechins/3000 mL) due to addition of catechin solution at 8.3 mL per minute for 10 minutes such that amount of catechins added per minute was 0.1% (3 g of catechins/3000 mL) and subsequent reaction for 5 minutes**

After 2000 g of purified water was placed in a reactor, citric acid and sodium bicarbonate were added to acid and alkali pumps of a fermenter, respectively, and then the pH was set to 5.0, which corresponds to an appropriate pH for enzyme treatment. Thereafter, 24 g of tannase was added to the reactor and activated by stirring at 200 rpm for 30 minutes at 40°C. For addition of green tea catechins to the reactor, 360 g of green tea catechins was dissolved in purified water to prepare 1 L of a green tea catechin solution. The green tea catechin solution thus prepared was added at a rate at which catechin was added at 3 g per minute (0.1% of the substrate) through a controlled-volume pump for 10 minutes, followed by reaction for 5 minutes, and such procedure was repeated. To investigate the enzyme inactivation inhibitory effect by the further addition of the substrate, the procedure of sampling the reaction solution every 15 minutes to measure the EGCG content was repeated for 3 hours to observe the change thereof over time. Even after catechin addition was completed, the change in EGCG content was observed for 3 hours.

As a result, as shown in FIG. 7, EGCG started to accumulate 1 hour and 30 minutes after the start of green tea catechin addition, and the EGCG content steadily increased by 3 hours, at which time the green tea catechin addition was ended. However, the EGCG content converged to zero 1 hour and 30 minutes after the ending of green tea catechin addition, and thus the degradation reaction was completed, and it was identified that the enzyme was not inactivated even after the ending of green tea catechin addition.

**Example 2-3: When total amount of catechins added were 20% (600 g of catechins/3000 mL) due to addition of catechin solution at 8.3 mL per minute for 5 minutes such that amount of catechin added per minute was 0.1% (3 g of catechins/3000 mL) and subsequent reaction for 10 minutes**

After 1400 g of purified water was placed in a reactor, citric acid and sodium bicarbonate were added to acid and alkali pumps of a fermenter, respectively, and then the pH was set to 5.0, which corresponds to an appropriate pH for enzyme treatment. Thereafter, 24 g of tannase was added to the reactor and activated by stirring at 200 rpm for 30 minutes at 40°C. For addition of green tea catechins to the reactor, 600 g of green tea catechins was dissolved in purified water to prepare 1.6 L of a green tea catechin solution. The green tea catechin solution thus prepared was added at a rate at which catechins were added at 3 g per minute (0.1% of the substrate) through a controlled-volume pump for 5 minutes, followed by reaction for 10 minutes, and such procedure was repeated. To investigate the enzyme inactivation inhibitory effect by the further addition of the substrate, the procedure of sampling the reaction solution every 30 minutes to measure the EGCG content was repeated for 10 hours to observe the change thereof over time. Even after catechin addition was completed, the change in EGCG content was observed for 30 minutes.

As a result, as shown in FIG. 8, EGCG started to accumulate 4 hours and 30 minutes after the start of substrate addition and the EGCG content steadily increased by 10 hours (20%), at which time the substrate addition was ended, and the complete inactivation of the enzyme was confirmed.

**Example 3: Measurement of enzymatic reaction completion time according to catechin content of green tea extract**

In order to investigate whether the method for producing an enzymatically treated green tea catechin product according to the present invention can be applied regardless of the catechin content of the green tea extract, the enzymatic reaction completion time was measured as follows.

**Example 3-1: Preparation of ethanol fraction of green tea extract**

After 5 kg of green tea leaves and 50 L of purified water were placed in a low-temperature concentration extractor, extraction was conducted at 80°C for 6 hours. The corresponding extract was filtered through a 5 $\mu$m filter and then concentrated to 25 L. Thereafter, 37.5 L of ethanol was added to 25 L of the concentrate, and fractionation was conducted three times. The separated ethanol layer was collected, concentrated, and dried, to yield about 1.1 kg of a dried product.

**Example 3-2: Preparation of ethyl acetate (EA) fraction of green tea extract**

After 5 kg of green tea leaves and 50 L of purified water were placed in a low-temperature concentration extractor, extraction was conducted at 80°C for 6 hours. The corresponding extract was filtered through a 5 $\mu$m filter and then concentrated to 25 L. Thereafter, 37.5 L of EA was added to 25 L of the solution, and fractionation was conducted three times. The separated EA layer was collected, concentrated, and dried, to give about 0.9 kg of a dried product.

**Example 3-3: Production of enzymatically treated green tea catechin product according to catechin content and measurement of enzymatic reaction completion time**

After 1 L of purified water was placed in a reactor, citric acid and sodium bicarbonate were added to acid and alkali pumps of a fermenter, respectively, and then the pH was set to 5.0. Thereafter, 24 g of tannase was placed in the reactor, and activated by stirring at 200 rpm for 30 minutes at 40°C. In order to equalize the amount of green tea catechins added per minute, 770 g of the ethanol fraction of the green tea extract prepared in Example 3-1, 430 g of the EA fraction of the green tea extract prepared in Example 3-2, and 360 g of Chinese catechins were dissolved in purified water to prepare 2 L of each green tea catechin solution. Each of the green tea catechin solutions thus prepared was added at 16.6 mL per minute through a controlled-volume pump for 5 minutes, followed by reaction for 10 minutes, and such procedure was repeated for 10 minutes. In order to investigate the enzymatic treatment completion time, sampling was conducted every 30 minutes after substrate addition to measure the EGCG content, and the reaction was determined as being completed when the EGCG converged to zero.

As a result, as shown in Table 2, each enzymatic reaction was completed within 7 hours regardless of the catechin content of the green tea extract.

TABLE 2

| Catechin type | Catechin content upon purification (%) | Total concentration of substrate (%) | Enzymatic reaction completion time (h) |
|---|---|---|---|
| Ethanol fraction of green tea extract | 34.5 | 26 | 7 |
| EA fraction of green tea extract | 62.1 | 14 | 6 |
| Chinese green tea catechins | 74.1 | 12 | 6 |

**Example 4: Measurement of maximum substrate treatment concentration according to enzyme concentration**

In order to investigate the change in maximum substrate treatment concentration according to the concentration of the enzyme used, the maximum substrate treatment concentration according to the enzyme concentration was measured as follows.

After 1 L of purified water was placed in a reactor, citric acid and sodium bicarbonate were added to acid and alkali pumps of a fermenter, respectively, and then the pH was set to 5.0. For the preparation of enzyme concentrations of 1 U/mL, 4 U/mL, 8 U/mL, 16 U/mL, and 32 U/mL based on the sum (3 L) of the volume of the green tea catechin solution to be added and the volume of the tannase solution, 6 g, 24 g, 48 g, 96 g, and 192 g of tannase (Tannase KTFH, 500 U/g) was separately added to the reactor, and the enzyme was activated by stirring at 200 rpm at 40°C for 30 minutes.

To further add green tea catechins to the reactor, 200 g, 400 g, 600 g, 800 g, and 1000 g of green tea catechins was dissolved in purified water to prepare 2 L of additional green tea catechin solutions. The substrate concentrations of the additional green tea catechin solutions correspond to 6%, 13%, 20%, 26%, and 33%, respectively, relative to the sum (3 L) of the volume of the green tea catechin solution to be added and the volume of the tannase solution.

In order to investigate the maximum substrate throughput according to the enzyme concentration, each of the additional green tea catechin solution prepared was added to each enzyme concentration such that the catechin solution was added at 16.6 mL per minute, through a controlled-volume pump for 5 minutes, followed by reaction for 10 minutes, and such procedure was repeated. Thereafter, in order to investigate the completion of the reaction, the reaction solution was sampled every 30 minutes to observe the change in EGCG content, and the reaction was determined as being completed when the EGCG content converged to zero. The enzyme reaction was carried out for each enzyme concentration and each substrate concentration, and if the reaction was completed, "o" was marked in Table 3.

As a result, as shown in Table 3, as the enzyme concentration increased, the maximum substrate treatment concentration also increased, but the substrate treatment concentration per enzyme unit (U/mL) decreased.

TABLE 3

| | | Substrate concentration (%) | | | | |
|---|---|---|---|---|---|---|
| | | 6 | 13 | 20 | 26 | 33 |
| Enzyme concentrati on, U/mL | 1 | ○ | - | - | - | - |
| | 4 | ○ | ○ | - | - | - |
| | 8 | ○ | ○ | ○ | - | - |
| | 16 | ○ | ○ | ○ | ○ | - |
| | 32 | ○ | ○ | ○ | ○ | ○ |

**Test Example 1: Analysis of active ingredients of enzymatically treated green tea catechin product**

In order to investigate the degradation of EGCG and ECG, the contents of active ingredients of the enzymatically treated green tea catechin products of Comparative Example 1 and Example 1 were measured.

Specifically, in order to measure the contents of EGCG, ECG, EGC, EC, and gallic acid, which are active ingredients of the enzymatically treated green tea catechin products of Comparative Example 1 and Example 1, high-performance liquid chromatography (Infinity 1260, Agilent, USA) was used, Poroshell 120EC-C18 (4.6 mm × 50 mm) was used as a column, and detection spectra were measured at UV 280 nm. The distilled water containing 0.1% phosphoric acid was used for mobile phase A, and 100% ACN was used for mobile phase B. Analysis was conducted under mobile phase conditions over time where mobile phase A was changed from 90% to 85% at 0-4 minutes and from 85% to 73% at 4-8 minutes, and the flow rate was 1 mL/min and the sample volume was 3 μL. As for the preparation of the standard, the active ingredients and 20 mg of a precursor standard were placed in a 50 mL flask, and dissolved in methanol to prepare a standard solution, which was then diluted for each concentration, followed by analysis, and then a calibration curve was made and the content of each of the active ingredients was measured.

As a result, HPLC chromatogram is shown in FIG. 9, and as shown in Table 4 below, EGCG and ECG in the enzymatically treated products of Comparative Example 1 and Example 1 were all converted into EGC, EC, and gallic acid by way of tannase treatment.

TABLE 4

| Contents of active ingredients | Gallic acid (mg/g) | EGC (mg/g) | EC (mg/g) | EGCG (mg/g) | ECG (mg/g) |
|---|---|---|---|---|---|
| Comparative Example 1 | 9.81 | 82.24 | 55.12 | 439.57 | 97.47 |
| Example 1 | 209.29 | 337.35 | 103.74 | N.D. | N.D. |
| N.D.: Not determined | | | | | |

**Test Example 2: Verification of muscle increase promoting effect by tannase treatment**

**Test Example 2-1: Identification of muscle cell differentiation promotion**

In order to investigate the ability to promote differentiation of muscle cells into muscle fibers by way of tannase treatment according to the method of the present invention, the muscle fiber diameter and length were measured.

Specifically, C2C12 muscle cells were distributed from the American Type Culture Collection (ATCC, Manassas, VA, USA). C2C12 muscle cells are a cell line that is widely used to study the metabolism of muscle cells, and the more active the differentiation of the cells, the more active the conversion into muscle fibers. The cell line received from the American Type Culture Collection (ATCC) was cultured under conditions in which Dulbecco's modified Eagle's medium (DMEM, Lonza, Allendale, NJ, USA) containing 20% fetal bovine serum (FBS, Gibco, Grand Island, NY, USA) and 1% penicillin-streptomycin (P/S, Gibco, Grand Island, NY, USA) was maintained at 5% $CO_2$ and 37°C. Stabilized C2C12 muscle cells were dispensed in a 24 well plate at a density of $1 \times 10^5$ cells/mL, cultured, and maintained until the cells were 80% confluent. The differentiation of muscle cells was induced by 2% horse serum (HS, Gibco, Grand Island, NY, USA) DEME for 2 days, and thereafter, the cells were cultured for 6 days with 2% HS DMEM changed every 2 days. Each culture was treated with the enzymatically converted green tea catechin extract at a concentration of 20 μL/mL during the differentiation of muscle cells, and on the 8th day when the differentiation was completed, the degree of differentiation of muscle cells was observed.

As a result, as shown in FIGS. 10 and 11, the muscle fiber diameter was 82.28 μm, and the muscle fiber length

was 1523.72 μm in the treatment with the enzymatically treated green tea catechin product of Example 1. In Comparative Example 1, the muscle fiber diameter was 82.28 μm, and the muscle fiber length was 1523.72 μm. Therefore, it was identified that muscle cells differentiated more actively when treated with the enzymatically treated green tea catechin product of Example 1. This indicates that the enzymatically treated green tea catechin product of Example 1 has a higher content of EC, which is effective in the treatment and prevention of senile muscular diseases.

**Test Example 2-2: Identification of expression of p-AMPK and muscle growth factor in C2C12 cells treated with enzymatically treated green tea catechin product**

In order to investigate the muscle increase effect of the enzymatically treated green tea catechin product according to the present invention, the AMPK activation and the expression of follistatin that regulates muscle growth were measured.

Specifically, C2C12 cells were seeded in a 6-well plate, cultured using DMEM containing 20% FBS, which was then changed with DMEM containing 2% HS to induce differentiation, and the cells were cultured by addition of the enzymatically treated green tea catechin product. After being harvested with SDS sample buffer, the protein lysate was obtained through sonication. In addition, 10% SDS-PAGE electrophoresis was performed, and proteins were transferred to the PVDF transfer membrane using a semi-dry transfer device. The transferred proteins were blocked with 5% skim milk for 1 hour at room temperature, and then incubated using total AMPK, phospho-AMPK (Thr172), and follistatin antibodies at 4°C overnight. The proteins were washed 3 times with TBS buffer containing 0.1% Tween-20, and then immunoblotted using anti-mouse HRP secondary antibody.

As a result, as shown in FIG. 12, strong p-AMPK and follistatin activities were confirmed in C2C12 cells treated with the enzymatically treated green tea catechin product of Example 1.

**Test Example 3: Identification of obesity prevention or treatment effect of enzymatically treated green tea catechin product**

**Test Example 3-1: Identification of inhibition of differentiation into adipose cells by enzymatically treated green tea catechin product**

In order to investigate the ability of the enzymatically treated green tea catechin product according to the present invention to inhibit precursor adipose cell proliferation, the lipid accumulation rate in precursor adipose cells was measured.

Specifically, 3T3-L1 precursor adipose cells were distributed from the American Type Culture Collection (ATCC, Manassas, VA, USA). The 3T3-L1 precursor adipose cells are a cell line that is widely used to study the metabolism of adipose cells, and the more active the differentiation of the cells, the more active the lipid accumulation in adipose cells. The cell line received from the American Type Culture Collection (ATCC) was cultured in conditions in which Dulbecco's modified Eagle's medium (DMEM, Lonza, Allendale, NJ, USA) containing 10% bovine calf serum (BCS, Gibco, Grand Island, NY, USA) and 1% penicillin-streptomycin (P/S, Gibco, Grand Island, NY, USA) was maintained at 5% $CO_2$ and 37°C. Stabilized 3T3-L1 precursor adipose cells were dispensed in a 24 well plate at a density of $1 \times 10^5$ cells/mL and cultured, and when the cells were 100% confluent, the cells were further maintained for two days. The adipose cell differentiation was induced for 2 days in 10% fetal bovine insulin (FBS, Gibco, Grand Island, NY, USA) DEME containing 0.5 mM IBMX (3-isobutyl-1-methylzanthine, Sigma, St. Louis, Mo, USA), 1 μM Dexamethasone (Sigma, St. Louis, MO, USA), and 10 μg/mL Insulin (Gibco, Grand Island, NY, USA), and after 2 days of culture, the cells were further cultured in 10% FBS DMEM containing 10 μg/mL insulin for 2 days. Thereafter, the cells were cultured for 4 days while the medium was changed with 10% FBS DMEM every two days. The culture was treated with the enzymatically treated green tea catechin products produced in Comparative Example 1 and Example 1 at concentrations of 25 μL/mL and 50 μL/mL during adipose cell differentiation, and on the 10th day when the differentiation was completed, the degree of adipose cell differential was observed.

In order to investigate whether the enzymatically treated green tea catechin products of Comparative Example 1 and Example 1 had the effects of inhibiting the differentiation of 3T3-L1 precursor adipose cells into adipose cells and inhibiting adipogenesis, Oil Red O staining that specifically stains triglycerides was conducted.

Specifically, the medium was eliminated from the cells obtained by the induction of adipocyte differentiation, and the cells were washed twice with phosphate buffered saline (PBS), and then fixed with 4% formaldehyde at room temperature for 30 minutes. After the fixation, the cells were washed with 60% isopropanol, and stained with 0.2% Oil Red O staining agent (dissolved in 60% isopropanol) at room temperature for 1 hour. After the staining, the cells were washed with distilled water, and then observed by an optical microscope. In addition, the stained cells were dissolved in isopropanol, and then the absorbance at 570 nm was measured using an ELIZA reader. The lipid accumulation rate in adipose cells was calculated using Expression 1.

Equation 1

lipid accumulation rate (%) = (absorbance of sample treatment group / absorbance of control group) × 100

As a result, as shown in FIG. 13, the enzymatically treated green tea catechin product of Example 1 showed, at a concentration of 50 μg/mL, an MDI triglyceride accumulation rate of 60%, which was lower than that of Comparative Example 1, with a statistically significant difference. It was therefore identified that the enzymatically treated green tea catechin product of the present invention efficiently inhibited the differentiation of precursor adipose cells into adipose cells and the generation of triglycerides, thereby exhibiting obesity prevention or treatment effects.

**Test Example 3-2: Identification of AMPK activation effect of enzymatically treated green tea catechin product in 3T3-L1 cells**

In order to investigate the body fat reduction effect of the enzymatically treated green tea catechin product according to the present invention, the AMPK activation efficacy was measured. The increase in AMPK activity in the energy metabolism is known to increase the phosphorylation of acetyl-CoA carboxylases 1 and 2 (ACCs).

Specifically, 3T3-L1 cells were seeded in a 6-well plate, cultured using DMEM containing 10% BSC, which was then changed with DMEM containing 1% FBS to induce differentiation, and then cultured by addition of the enzymatically treated green tea catechin product. After being harvested with SDS sample buffer, the protein lysate was obtained through sonication. In addition, 10% SDS-PAGE electrophoresis was performed, and proteins were transferred to the PVDF transfer membrane by using a semi-dry transfer device. The transferred proteins were blocked with 5% skim milk for 1 hour at room temperature, and then incubated using total AMPK and phospho-AMPK (Thr172) antibodies at 4°C overnight. The proteins were washed 3 times with TBS buffer containing 0.1% tween-20, and then immunoblotted using anti-mouse HRP secondary antibody.

As a result, as shown in FIG. 14, strong p-AMPK activity was confirmed in the cells treated with the enzymatically treated green tea catechin product of Example 1 compared with the control group and Comparative Example 1.

**Test Example 3-3: Identification of conversion of white adipose tissue into brown adipose tissue by enzymatically treated green tea catechin product in 3T3-L1 cells**

In order to investigate the body fat reduction effect of the enzymatically treated green tea catechin product according to the present invention, the activities of UCP1 and PRDM16 that cause the browning of white adipose tissue were measured. Adipose tissues may be classified into white adipose tissue that conducts adipose storage as a main function and brown adipose tissue that conducts heating through adipose burning as a main function. The browning of white adipose tissue has been reported to produce obesity treatment effects and energy metabolic function normalization effects.

Specifically, 3T3-L1 cells were seeded in a 6-well plate, cultured using DMEM containing 10% BSC, which was then changed with DMEM containing 1% FBS to induce differentiation, and then cultured by addition of the enzymatically treated green tea catechin product. After the cells were harvested with TRI reagent, the RNA lysate was obtained through sonication. UCP1 and PRDM16 primers were attached to the extracted RNA, followed by real-time polymerase chain reaction, and RNA expression was measured.

As a result, as shown in FIG. 15, UCP1 and PRDM16 were highly expressed in the cells treated with the enzymatically treated green tea catechin product of Example 1 compared with Comparative Example 1.

**Test Example 3-4: Identification of body weight loss effect of enzymatically treated green tea catechin product**

In order to investigate the body weight loss effect of the enzymatically treated green tea catechin product according to the present invention, the change in body weight was measured for animal models having obesity induced by a high-fat diet.

Specifically, the test was carried out on male C57BL/6 mice. 5-week-old C57BL/6 mice were purchased from Orient Bio (Co.) and used. After quarantine and acclimation for one week, healthy animals without weight loss were selected and used for the test. The test animals were raised in a breeding environment set at a temperature of 23±3°C, a relative humidity of 50±10%, a ventilation frequency of 10 to 15 times/hour, a lighting time of 12 hours, and an illuminance of 150 Lux to 300 Lux. Throughout the entire period of the test, the test animals were free to consume a solid feed (Cargill Agri Purina Co., Ltd.) and drinking water. After 1-week acclimation, healthy animals were selected, and classified into Control group 1, Control group 2, Treatment group 1, Treatment group 2, and Treatment group 3, according to the randomized block design, and ten test animals were used for each test group. The treatment groups were orally administered the enzymatically treated green tea catechin product of Example 1 in an amount of 50 mg/kg, 100 mg/kg, or 200 mg/kg using a feeding needle for mice. The control groups were orally administered only pure water and set as medium control groups. Throughout the entire period of the test, the control diet and high-fat diet groups were fed a control diet (energy ratio kcal%; protein:carbohydrate:fat = 20:70:10) and a high-fat diet (energy ratio kcal%; protein:carbohydrate:fat = 20:20:60) purchased from Research Diets, Inc. (New Brunswick, NJ, USA), respectively, and were free to consume diets and drinking water. The diet compositions of the control diet and the high-fat diet are shown in Table 5. The test material dissolved in physiological saline was orally administered for 8 weeks, and Control group 1 and Control group 2 were orally administered physiological saline containing no test material in the same manner as the other test groups. All the animals were measured for body weight once a week immediately before administration at the time of starting administration and during the test period. After ending of administration for 8 weeks, the change in body weight was calculated. The food efficiency ratio (FER) was obtained by dividing the weight gain during the test period by the amount of food consumed during the same period, and the total food efficiency ratio was measured by quantification.

TABLE 5

| Composition of diets for testing (g/kg diet) | | | | |
|---|---|---|---|---|
| | Control diet (10 kcal% fat) | | High-fat diet (60 kcal% fat) | |
| | g | kcal | g | kcal |
| Casein, 80 Mesh | 200 | 800 | 200 | 800 |
| L-Cystine | 3 | 12 | 3 | 12 |
| Corn starch | 315 | 1,260 | 0 | 0 |
| Maltodextrin 10 | 35 | 140 | 125 | 500 |
| Sucrose | 350 | 1,400 | 68.8 | 275 |
| Cellulose | 50 | 0 | 50 | 0 |
| Soybean oil | 25 | 225 | 25 | 225 |
| Lard* | 20 | 180 | 245 | 2,205 |
| Mineral mix | 10 | 0 | 10 | 0 |
| Dicalcium phosphate | 13 | 0 | 13 | 0 |
| Calcium carbonate | 5.5 | 0 | 5.5 | 0 |
| Potassium citrate, $1H_2O$ | 16.5 | 0 | 16.5 | 0 |
| Vitamin mix | 10 | 40 | 10 | 40 |
| Choline bitartrate | 2 | 0 | 2 | 0 |
| Total | 1,055 | 4,057 | 773.8 | 4,057 |

As a result, as shown in Table 6 and FIG. 16, the body weight was rapidly increased in Control group 2 compared with Control group 1, while the body weight gains in Treatment groups 1, 2, and 3 were similar to that in Control group 1 after one week of feeding.

As a result of digitalizing the results and comparing the results among the respective groups, as shown in Table 6 below, the body weight gain was halved due to the administration of Example 1, confirming that body weight gain was suppressed. The food efficiency ratio (body weight gain compared with intake) was increased by about 3-fold in the high-fat diet group compared with the normal diet group, and the food efficiency ratios of Treatment group 3 and Comparative group 1 showing smallest total weight gains were significantly lower those of the other groups. The food efficiency ratio of mice treated with 300 mg/kg Comparative Example 1 was similar to that of mice treated with 200 mg/kg Example 1, confirming that the body weight loss effect of the enzymatically treated green tea catechin product produced by way of the method of the present invention was greater.

TABLE 6

| | | Initial body weight (g) [1] | Final body weight (g) [1] | Body weight gain (g) [1] | FER (%) [2] |
|---|---|---|---|---|---|
| Control group 1 | Normal diet | 20.5±0.4 | 27.6±0.6 | 7.1±0.8 | 0.051±0.006 |
| Control group 2 | High-fat diet | 20.9±0.3 | 40.9±0.8[###] | 20.1±1.0[###] | 0.149±0.009[###] |
| Treatment group 1 | Example 1 50 mg/kg | 20.6±0.4 | 34.7±0.8*** | 14.1±0.9*** | 0.115±0.007*** |
| Treatment group 2 | Example 1 100 mg/kg | 20.6±0.4 | 33.4±1.2*** | 12.9±0.9*** | 0.109±0.007*** |
| Treatment group 3 | Example 1 200 mg/kg | 20.8±0.4 | 31.1±0.7*** | 10.3±0.5*** | 0.089±0.004*** |
| Comparative group 1 | Comparative Example 1300 mg/kg | 20.6±0.3 | 30.9±1.2*** | 10.2±1.0*** | 0.095±0.007*** |
| [1] Significant (t-Test): * $p < 0.05$, ** $p < 0.01$, *** $p < 0.001$ *vs.* control group 1 Significant (t-Test): # $p < 0.05$, ## $p < 0.01$, ### $p < 0.001$ vs. control group 2 [2] FER (%); Feed efficiency ratio, Body weight gain (g) / Food intake (g) | | | | | |

While the present invention has been described with reference to the particular illustrative embodiments, a person skilled in the art to which the present invention pertains can understand that the present invention may be embodied in other specific forms without departing from the technical spirit or essential characteristics thereof. Therefore, the embodiments described above should be construed as being exemplified and not limiting the present disclosure. The scope of the present invention is not defined by the detailed description as set forth above but by the accompanying claims of the invention, and it should also be understood that all changes or modifications derived from the definitions and scopes of the claims and their equivalents fall within the scope of the invention.

**Claims**

1. A method for producing an enzymatically treated catechin product, the method comprising:

   a first step of preparing a tannase solution;
   a second step of adding a catechin solution to the tannase solution;
   a third step of reacting catechins and tannase contained in the solutions in the second step; and
   a fourth step of continuously repeating the second step and the third step.

2. The method of claim 1, wherein the catechins are contained in a green tea extract or a fraction thereof.

3. The method of claim 2, wherein the content of catechins in the green tea extract and the fraction thereof is 1% to 99%.

4. The method of claim 1, wherein the concentration of tannase in the first step is 1 U/mL to 50 U/mL relative to the volume of the total solution, which is the sum of the volume of the tannase solution in the first step and the volume of the catechin solution added in the second step.

5. The method of claim 1, wherein the amount of catechins added per minute in the second step is 0.01% (w/v) to 20% (w/v) relative to the volume of the total solution, which is the sum of the volume of the tannase solution in the first step and the volume of the catechin solution added in the second step.

6. The method of claim 1, wherein the total amount of catechins added in the second step is 0.01% (w/v) to 40% (w/v) relative to the volume of the total solution, which is the sum of the volume of the tannase solution in the first step and the volume of the catechin solution added in the second step.

7.  The method of claim 1, wherein the third step is performed for 1 to 60 minutes.

8.  The method of claim 1, wherein the second step and the third step are continuously repeated until the amount of catechins added in the second step is 1% (w/v) to 90% (w/v) relative to the volume of the total solution, which is the sum of the volume of the tannase solution in the first step and the volume of the catechin solution added in the second step.

9.  The method of claim 1, further comprising: inactivating tannase; and/or filtering, concentrating, and/or drying the reaction solution.

10. The method of any one of claims 1 to 9, wherein the enzymatically treated catechin product contains less than 1 part by weight of the sum of epigallocatechin gallate (EGCG) and epicatechin gallate (ECG) relative to 100 parts by weight of the sum of gallic acid, epigallocatechin gallate (EGCG), epicatechin gallate (ECG), epigallocatechin (EGC), and epicatechin (EC).

11. A pharmaceutical composition for preventing or treating obesity or sarcopenia, the composition comprising an enzymatically treated catechin product produced by way of the method of claim 10.

12. A food composition for preventing or alleviating obesity or sarcopenia, the composition comprising the enzymatically treated catechin product produced by way of the method of claim 10.

13. Use of the enzymatically treated catechin product produced by way of the method of claim 10 for preventing or treating obesity or sarcopenia.

14. A method for preventing or treating obesity or sarcopenia in a subject in need thereof, the method comprising administering to the subject the enzymatically treated catechin product produced by way of the method of claim 10.

FIG. 1

Epicatechin gallate = Epicatechin + Gallic acid

Epigallocatechin gallate = Epigallocatechin + Gallic acid

FIG. 2

```
┌─────────────────────────────┐
│      Green tea catechins     │
└─────────────────────────────┘
              │
              ▼
┌─────────────────────────────┐         ┌─────────────────────────────────┐
│  Prepare green tea catechin  │◄────────│          Add tannase            │
│      reaction solution       │         └─────────────────────────────────┘
└─────────────────────────────┘
              │
              ▼
┌─────────────────────────────┐         ┌─────────────────────────────────┐
│ Continously treat with       │◄────────│  pH adjuster (alkalizing agent) │
│ additional green tea catechin│         └─────────────────────────────────┘
│ solution and secure reaction │
│ time                         │
└─────────────────────────────┘
              │
              ▼
┌─────────────────────────────┐
│     Inactivate enzyme        │
│      (80°C, 30 min)          │
└─────────────────────────────┘
              │
              ▼
┌─────────────────────────────┐
│         Concentrate          │
└─────────────────────────────┘
              │
              ▼
┌─────────────────────────────┐
│            Dry               │
└─────────────────────────────┘
```

FIG. 3

FIG. 4

FIG. 5

FIG. 6

FIG. 7

FIG. 8

FIG. 9

FIG. 10

Muscle fiber diamter

FIG. 11

## Muscle fiber length

FIG. 12

FIG. 13

FIG. 14

p-AMPK

AMPK

Control          Comparative          Example 1
                 Example 1

FIG. 15

FIG. 16

**Body Weight**

## INTERNATIONAL SEARCH REPORT

| International application No. |
|---|
| PCT/KR2020/002940 |

| A. CLASSIFICATION OF SUBJECT MATTER |
|---|
| *A61K 36/82(2006.01)i, A61K 31/192(2006.01)i, A61K 31/353(2006.01)i, A61P 3/04(2006.01)i, A61P 21/00(2006.01)i, A23L 33/105(2016.01)i, A23L 29/00(2016.01)i* |
| According to International Patent Classification (IPC) or to both national classification and IPC |

| B. FIELDS SEARCHED |
|---|
| Minimum documentation searched (classification system followed by classification symbols) |
| A61K 36/82; A23F 3/06; A23L 3/3508; C09K 15/08; A61K 31/192; A61K 31/353; A61P 3/04; A61P 21/00; A23L 33/105; A23L 29/00 |
| Documentation searched other than minimum documentation to the extent that such documents are included in the fields searched |
| Korean utility models and applications for utility models: IPC as above |
| Japanese utility models and applications for utility models: IPC as above |
| Electronic data base consulted during the international search (name of data base and, where practicable, search terms used) |
| eKOMPASS (KIPO internal) & Keywords: tannase, catechin, green tea, enzyme, obesity, sarcopenia |

| C. DOCUMENTS CONSIDERED TO BE RELEVANT | | |
|---|---|---|
| Category* | Citation of document, with indication, where appropriate, of the relevant passages | Relevant to claim No. |
| X | ROBERTO, B. S. et al. Immobilized tannase treatment alters polyphenolic composition in teas and their potential anti-obesity and hypoglycemic activities in vitro. Food & Function. 2016, vol. 7, pages 3920-3932 See abstract; page 3922, left column; page 3929, left column. | 1-3 |
| Y | | 4-13 |
| Y | 홍양희 등. Tannase를 이용한 녹차의 생물학적 전환의 최적 조건 마련 및 라디칼 소거능. 한국식품영양과학회지. 2011, vol. 40, no. 11, pages 1501-1506 (HONG, Yang Hee et al. Optimal Reaction Conditions and Radical Scavenging Activities for the Bioconversion of Green Tea Using Tannase. Journal of the Korean Society of Food Science and Nutrition.) See pages 1502-1505; figures 1-3. | 4-13 |
| A | JP 2015-002725 A (LETS KK.) 08 January 2015 See the entire document. | 1-13 |
| A | JP 2006-083352 A (HASEGAWA T CO., LTD.) 30 March 2006 See the entire document. | 1-13 |
| A | 김동호 등. 단닌분해효소를 이용한 녹차 농축액의 품질 변화. 한국식품영양학회지. 2011, vol. 24, no. 4, pages 720-724 (KIM, Dong-ho et al. Changes in the Quality of Green Tea Concentration through Tannase Treatment. The Korean Journal of Food and Nutrition.) See the entire document. | 1-13 |

☐ Further documents are listed in the continuation of Box C.　　☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 30 JUNE 2020 (30.06.2020) | 30 JUNE 2020 (30.06.2020) |

| Name and mailing address of the ISA/KR | Authorized officer |
|---|---|
| Korean Intellectual Property Office Government Complex Daejeon Building 4, 189, Cheongsa-ro, Seo-gu, Daejeon, 35208, Republic of Korea | |
| Facsimile No. +82-42-481-8578 | Telephone No. |

Form PCT/ISA/210 (second sheet) (January 2015)

## INTERNATIONAL SEARCH REPORT

| International application No. |
| --- |
| **PCT/KR2020/002940** |

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 14
   because they relate to subject matter not required to be searched by this Authority, namely:

   Claim 14 pertains to a method for treatment of the human body by surgery or therapy, as well as a diagnostic method (PCT Article 17(2)(a)(i) and PCT Rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

International application No.

**PCT/KR2020/002940**

| Patent document cited in search report | Publication date | Patent family member | Publication date |
|---|---|---|---|
| JP 2015-002725 A | 08/01/2015 | None | |
| JP 2006-083352 A | 30/03/2006 | JP 4443359 B2 | 31/03/2010 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020070019395
- KR 100891393
- KR 1020180009938
- KR 1020120021407

**Non-patent literature cited in the description**

- **ANJALI PANDEY et al.** *Advances in Research,* 2014, vol. 2 (10), 556-570